# EUROPEAN PATENT SPECIFICATION

(11) **EP 2 895 185 B1**
(45) Date of publication and mention of the grant of the patent: **10.07.2019**
(21) Application number: 13837673.6
(22) Date of filing: 13.09.2013
(51) Int. Cl.: A61K 38/15, A61K 31/661, A61K 38/18, A61K 38/24, A61P 15/08

(54) **STIMULATION OF OVARIAN FOLLICLE DEVELOPMENT AND OOCYTE MATURATION**
STIMULIERUNG VON OVARIALFOLLIKELENTWICKLUNG UND OOZYTENREIFUNG
STIMULATION DU DÉVELOPPEMENT DE FOLLICULE OVARIEN ET DE LA MATURATION D'OVOCYTE

(30) Priority: 13.09.2012 US 201261700799 P; 12.03.2013 US 201361778024 P
(43) Date of publication of application: 22.07.2015
(73) Proprietor: The Intellectual Property Management Center in the St. Mariana University School of Medicine, Kawasaki City Kanagawa 216-8511 (JP); The Board of Trustees of the Leland Stanford Junior University, Stanford CA 94305-2038 (US)
(72) Inventor: HSUEH, Aaron J.W., Stanford, California 94305 (US); CHENG, Yuan, Palo Alto, California 94301 (US); DEGUCHI, Masashi, Kobe Hyogo 658-0047 (JP); KAWAMURA, Kazuhiro, Akita City Akita 010-08543 (JP)
(74) Representative: Mewburn Ellis LLP
(86) International application number: PCT/US2013/059800
(87) International publication number: WO 2014/043568

(56) References cited:
- WO-A1-2013/090787
- WO-A1-2013/188138
- US-A1- 2003 157 086
- US-A1- 2010 191 040
- US-A1- 2011 015 169
- MARY B ZELINSKI ET AL: "In vivo delivery of FTY720 prevents radiation-induced ovarian failure and infertility in adult female nonhuman primates", FERTILITY AND STERILITY, ELSEVIER SCIENCE INC, NEW YORK, NY, USA, vol. 95, no. 4, 4 January 2011 (2011-01-04), pages 1440-1445.e7, XP028169836, ISSN: 0015-0282, DOI: 10.1016/J.FERTNSTERT.2011.01.012 [retrieved on 2011-01-10]
- GARDELL S E ET AL: "Emerging medicinal roles for lysophospholipid signaling", TRENDS IN MOLECULAR MEDICINE, ELSEVIER CURRENT TRENDS, GB, vol. 12, no. 2, 1 February 2006 (2006-02-01), pages 65-75, XP028058700, ISSN: 1471-4914, DOI: 10.1016/J.MOLMED.2005.12.001 [retrieved on 2006-02-01]
- POLESELLO ET AL.: 'Salvador-Warts-Hippo Signaling Promotes Drosophila Posterior Follicle Cell Maturation Downstream of Notch.' CURRENT BIOLOGY vol. 17, 06 November 2007, pages 1864 - 1870, XP022336825
- GENEVET ET AL.: 'The Hippo pathway regulates apical-domain size independently of its growth- control function.' JOUMAL OF CELL SCIENCE vol. 122, 2009, pages 2360 - 2370, XP055249048
- HALDER ET AL.: 'Hippo signaling: growth control and beyond.' DEVELOPMENT vol. 138, 2011, pages 9 - 22, XP055249062
- KAWAMURA ET AL.: 'Hippo signaling disruption and AM stimulation of ovarian follicles for infertility treatment' PROC NATI ACAD SCI USA vol. 110, no. 43, 22 October 2013, pages 17474 - 17479, XP055249063

## Description

### FEDERALLY SPONSORED RESEARCH AND DEVELOPMENT

This invention was made with Government support under grant HD068158 awarded by the National Institutes of Health. The Government has certain rights in this invention.

### Background of the Invention

The growth and maturation of mammalian germ cells is intricately controlled by hormones; including gonadotropins secreted by the anterior pituitary; and local paracrine factors. The majority of the oocytes within the adult human ovary are maintained in prolonged stage of first meiotic prophase; enveloped by surrounding follicular somatic cells. Periodically, a group of primordial follicles enters a stage of follicular growth. During this time, the oocyte undergoes a large increase in volume, and the number of follicular granulosa cells increase. The maturing oocyte synthesizes paracrine factors that allow the follicle cells to proliferate, and the follicle cells secrete growth and differentiation factors that enhance angiogenesis and allow the oocyte to grow. After progressing to a certain stage, oocytes and their follicles die, unless they are exposed to gonadotropic hormones that prevent somatic cell apoptosis.

Mammalian ovaries consist of follicles as basic functional units. The total number of ovarian follicles is determined early in life, and the depletion of this pool leads to reproductive senescence. Each follicle develops to either ovulate or to undergo degeneration. Individual follicles consist of an innermost oocyte, surrounding granulosa cells, and outer layers of thecal cells. The fate of each follicle is controlled by endocrine as well as paracrine factors. The follicles develop through primordial, primary, and secondary stages before acquiring an antral cavity. At the antral stage a few follicles, under the cyclic gonadotropin stimulation that occurs after puberty, reach the preovulatory stage and become a major source of the cyclic secretion of ovarian estrogens in women of reproductive age. In response to preovulatory gonadotropin surges during each reproductive cycle, the dominant Graafian follicle ovulates to release the mature oocyte for fertilization, whereas the remaining theca and granulosa cells undergo transformation to become the corpus luteum.

Once entering the growing pool, ovarian follicles continue to progress into primary, secondary, and early antral stages with minimal loss. Although FSH treatment is widely used to generate preovulatory follicles in infertile patients mainly by suppressing the apoptosis of early antral follicles, some patients are low responders to FSH treatment because their ovaries contain few early antral follicles as reflected by their elevated serum FSH and lower AMH levels on day 3 of the menstrual cycle.

Throughout the reproductive life, primordial follicles undergo initial recruitment to enter the growing pool of primary follicles. In the human ovary, it is estimated that greater than 120 days are required for the primary follicles to reach the secondary follicle stage, whereas it is estimated that 71 days are needed to grow from the secondary to the early antral stage. Once initiated to enter the growing pool, ovarian follicles progress to reach the antral stage and minimal follicle loss was found until the early antral stage. During cyclic recruitment, increases in circulating FSH allow a cohort of antral follicles to escape apoptotic demise. Among this cohort, a leading follicle emerges as dominant by secreting high levels of estrogens and inhibins to suppress pituitary FSH release. The result is a negative selection of the remaining cohort, leading to its ultimate demise. Concomitantly, increases in local growth factors and vasculature allow a positive selection of the dominant follicle, thus ensuring its final growth and eventual ovulation and luteinization. After cyclic recruitment, it takes only 2 weeks for an antral follicle to become a dominant Graafian follicle. The overall development of human follicles from primordial to preovulatory stages require more than six months.

The development of follicles from the smallest primordial and primary follicles to the largest preovulatory follicles requires different stage-dependent stimulatory and survival factors. Methods of efficiently maturing ovarian follicles from primary through secondary, antral, and preovulatory stages is of great interest, including methods for *in vitro* and *in vivo* follicle maturation. The present invention addresses this issue.

### SUMMARY OF THE INVENTION

Aspects of the invention are set out in the claims. Compositions and methods are provided for promoting the growth of mammalian ovarian follicles to a pre-ovulatory stage by contacting the follicles with an effective dose of at least one of an agent that disrupts signaling in the Hippo pathway, or an agent that acts downstream of disrupted Hippo signaling, for a period of time sufficient to grow follicles to a pre-ovulatory state. The contacting may be performed in the absence of physical disruption of the ovary, i.e. the ovary is intact. In some disclosure herein the ovarian follicles are contacted with the agent in an *ex vivo* culture. In other disclosure herein the ovarian follicles are contacted with the agent *in vivo.* Where the contacting is performed *in vivo,* the agent may be administered locally to the ovary, e.g. to women suffering from premature ovarian failure, women suffering from polycystic ovarian syndrome, middle-aged infertile women, etc. The effective dose is a dose that allows ovarian follicles to undergo sufficient growth to reach the pre-ovulatory stage.

Agents that disrupt signaling in the Hippo pathway include agents that polymerize, or stabilize polymerized, actin. Such agents include known drugs, including without limitation the cyclic peptide jasplakinolide (JASP); and sphingosine 1-phosphate (S1P) receptor modulator, e.g. S1P, FTY720, lysophosphatidic acid (LPA) *etc.* Agents that act downstream of disrupted Hippo signaling include the CCN growth factor proteins, e.g. CCN 2, 3, 5 or 6.

The methods of the disclosure may be further combined with the step of contacting the ovarian follicles with additional agents that activate growth of ovarian follicles, including without limitation contacting the follicles with at least one of a phosphatase and tensin homolog (PTEN) inhibitor, and a phosphatidylinositol 3-kinase (Pl3 kinase) activator, which provides for an additive effect.

In some disclosure herein, the exposure is performed *in vitro, e.g.* in an organ or tissue culture, where at least one ovarian follicle is exposed to an effective dose of at least one of an agent that disrupts signaling in the Hippo pathway, or an agent that acts downstream of disrupted Hippo signaling. The treated follicle may be utilized for *in vitro* purposes, for example for *in vitro* fertilization, generation of embryonic stem cells, *etc.,* or may be transplanted to provide for *in vivo* uses. Transplantation modes of interest include, without limitation, transplantation of one or more follicles, including follicles present in an ovary that has not been physically disrupted, to a kidney capsule, to a subcutaneous site, near the fallopian tubes, to an ovarian site, e.g. where one ovary has been retained and one has been removed for *ex vivo* treatment, the one or more treated follicles may be transplated to the site of the remaining ovary.

In some disclosure herein, *in vitro* treatment is followed by ovarian transplantation to activate follicles for the generation of preovulatory oocytes, which may be followed by *in vitro* or *in vivo* fertilization.

Individuals of interest include endangered species, economically important animals, women suffering from premature ovarian failure, women suffering from polycystic ovarian syndrome, middle-aged infertile women, follicles derived from human embryonic stem cells and primordial germ cells, and the like. In other disclosure herein, the exposure is performed *in vivo,* locally, e.g. by intra-ovarian injection, or systemically administered to an individual.

Following exposure of an individual to an effective dose of at least one of an agent that disrupts signaling in the Hippo pathway, or an agent that acts downstream of disrupted Hippo signaling, the individual may be treated with follicular stimulating hormone (FSH) or FSH analogs, including recombinant FSH, naturally occurring FSH in an *in vivo* host animal, FSH analogs, e.g. FSH-CTP, pegylated FSH, and the like, at a concentration that is effective to initiate follicular growth.

Where the follicles have been stimulated to the pre-ovulatory stage stage, the individual may be treated lutenizing hormone (LH) or an agonist thereof, which agonists specifically include chorionic gonadotropins, e.g. equine chorionic gonadotropin (eCG), human chorionic gonatotropin (HCG), *etc.,* at an ovulatory dose. In addition, the follicles may be exposed *in vivo* or *in vitro* to one or more of c-kit ligand, neurotrophins, vascular endothelial growth factor (VEGF), bone morphogenetic protein (BMP)-4, BMP7, leukemia inhibitory factor, basic FGF, keratinocyte growth factor; and the like.

The period of time effective for stimulation with an effective dose of at least one of an agent that disrupts signaling in the Hippo pathway, or an agent that acts downstream of disrupted Hippo signaling according to the methods of the disclosure is usually at least about one hour and not more than about 5 days, and may be at least about 12 hours and not more than about 4 days, e.g. 2, 3, 4 or 5 days.

### BRIEF DESCRIPTION OF THE FIGURES

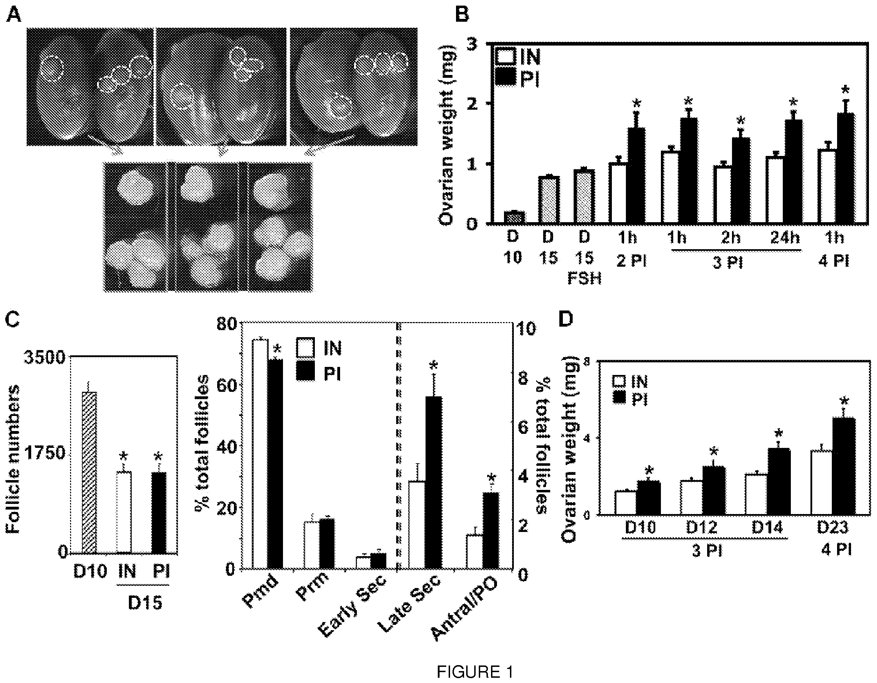

Fig. 1. Ovarian fragmentation and grafting promoted follicle growth in mice. Paired ovaries from juvenile mice were grafted into kidneys of adult ovariectomized mice (intact: IN; pieces: PI). Hosts were injected with FSH daily for 5 days before graft retrieval. A, Morphology of paired ovarian grafts with or without fragmentation into 3 pieces. Upper panel: grafts inside kidney capsules; Lower panel: isolated paired grafts. B, Weights of paired ovaries following fragmentation into 2 to 4 pieces from day 10 (D10) mice and incubated for 1 to 24h before grafting. Ovarian weights before grafting (D10) and at 5 days after grafting with (D15 FSH) or without FSH treatment (D15) served as controls. n= 8-22. C, Follicle dynamics before and after grafting of intact and fragmented (3 pieces) ovaries from day 10 mice. Left: total follicle numbers, Right: follicle dynamics. n=5. Pmd=primordial; Prm=primary, Sec=secondary; PO=preovulatory. D, Weights of paired ovaries from mice at different ages following fragmentation into 3-4 pieces and grafting. Mean±SEM; * p<0.05. n=8-22.

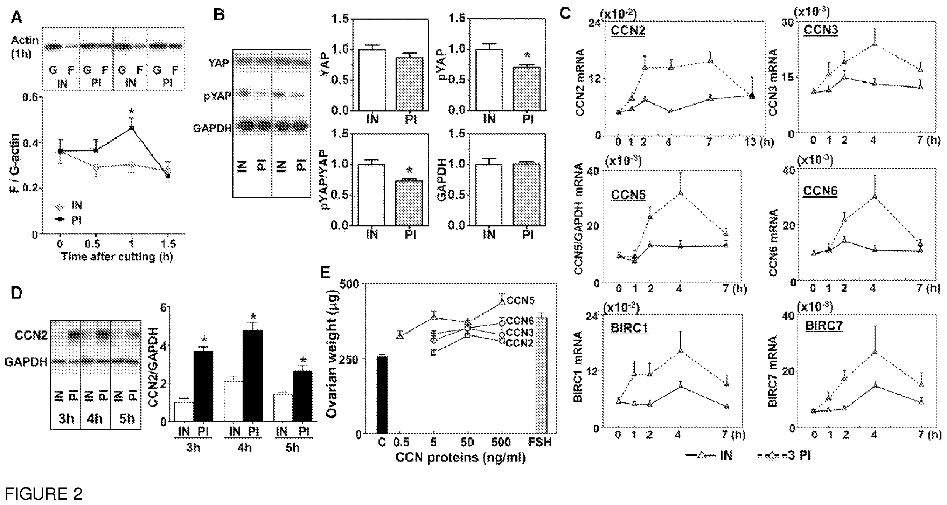

Fig. 2. Fragmentation of murine ovaries increased actin polymerization, disrupted Hippo signaling, and increased CCN growth factors and apoptosis inhibitors. A, Ovarian fragmentation increased F-actin levels. Paired ovaries from day 10 mice were cut into 3 pieces or kept intact before immunoblotting analyses of F- and G-actin levels (upper panel). Lower panel: F- to G-actin ratios. N=6-11. B, Ovarian fragmentation decreased phospho-YAP(pYAP) levels and pYAP to total YAP ratios. Paired ovaries with or without cutting were incubated for 1h, followed by immunoblotting. Left panel: representative immunoblots; Right panel: ratios of different antigens. n=8 pairs. C, Ovarian fragmentation increased expression of CCN growth factors and BIRC apoptosis inhibitors. Paired ovaries with or without cutting were incubated for 1h with subsequent grafting before analyses of transcript levels normalized by GAPDH. Intact ovaries: solid lines; pooled 3 pieces: dashed lines. n=10-15. D, Ovarian fragmentation increased CCN2 proteins. Paired ovaries with or without cutting were incubated for 3-5h before immunoblotting. Upper panel: representative blots; lower panel: quantitative analyses. N=3-5. E, Treatment with CCN2, 3, 5, and 6 increased ovarian explant weights. Explants from day 10 mice were cultured with different CCN growth factors for 4 days before weighing. n=5-6. Mean±SEM; * p<0.05. IN=Intact; PI=Pieces.

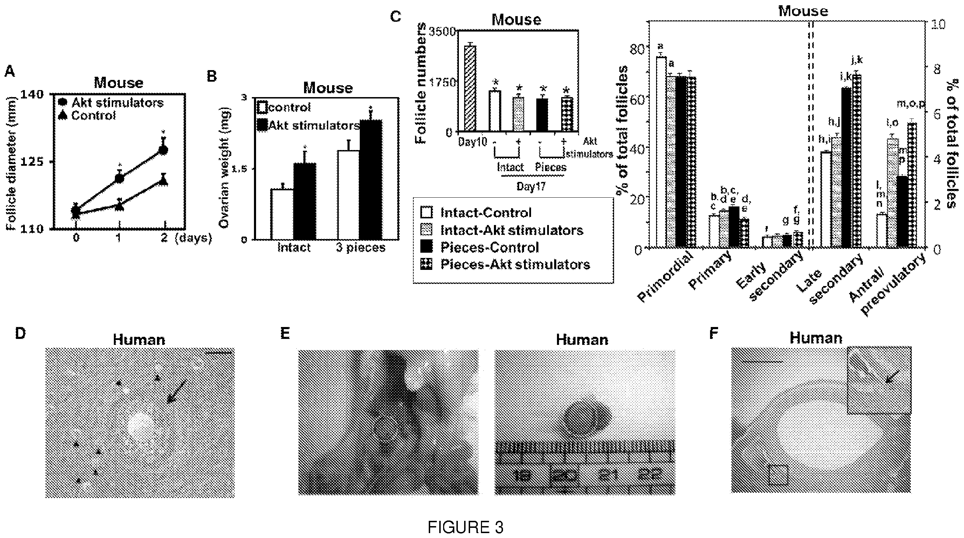

Fig. 3. Additive effects of Hippo signaling disruption and Akt stimulation promoted secondary follicle growth. A) Secondary follicles are responsive to IVA treatment. Secondary follicles (115 um in diameter) were isolated from mice at day 10 of age and cultured in vitro with the standard IVA protocol (first day with bPV(hopic) at 3 mM and 740YP at 15 ug/ml, followed by one more day with 740YP alone), followed by treatment with daily FSH (100 ng/ml) for two more days (IVA +FSH). The control groups were either treatment with media alone for four days or treated with the IVA reagent for two days followed by media alone (IVA alone). B) Ovarian fragmentation and IVA treatment led to additive effects on ovarian weight gain and secondary follicle growth. Paired ovaries from mice at day 10 of age were fragmented into three pieces. Fragments from one side were treated with the IVA protocol (bpV(hopic) at 30 uM and 740YP at 150 ng/ml for one day, followed by one more day with 740YP only) whereas fragments from the contralateral side were incubated in media alone. Ovarian fragments were then allo-transplanted into kidney capsules of adult ovariectomized mice with hosts injected with FSH (daily at 1 IU/animal). Five 5 days later, ovarian grafts were retrieved, fixed and weighted. Left panel: graft weights; right panel: Follicle dynamics of grafted ovarian fragments. Percentages of follicles at different stages were determined based on serial sections of grafts. C, Follicle dynamics before and after grafting of intact and fragmented murine ovaries with or without treatment with Akt stimulators. Left: total follicle numbers, Right: follicle dynamics. n=4. Same letter symbols indicate significant differences (p<0.05). D-F. Vitrified human cortical strips were thawed and fragmented into cubes before treatment with Akt stimulators for 2 days followed by grafting into immune-deficient mice for 4 weeks. D. Cortical strips before grafting. Arrow: a secondary follicle, arrowheads: primordial/primary follicles. Scale bar, 100 µm. E. A kidney graft in situ (left) and after isolation (right), showing an antral follicle. F. Histology of two large antral follicles with the side view of one showing an oocyte at the germinal vesicle stage (arrow). Scale bar, 1 mm. Mean±SEM; * p<0.05.

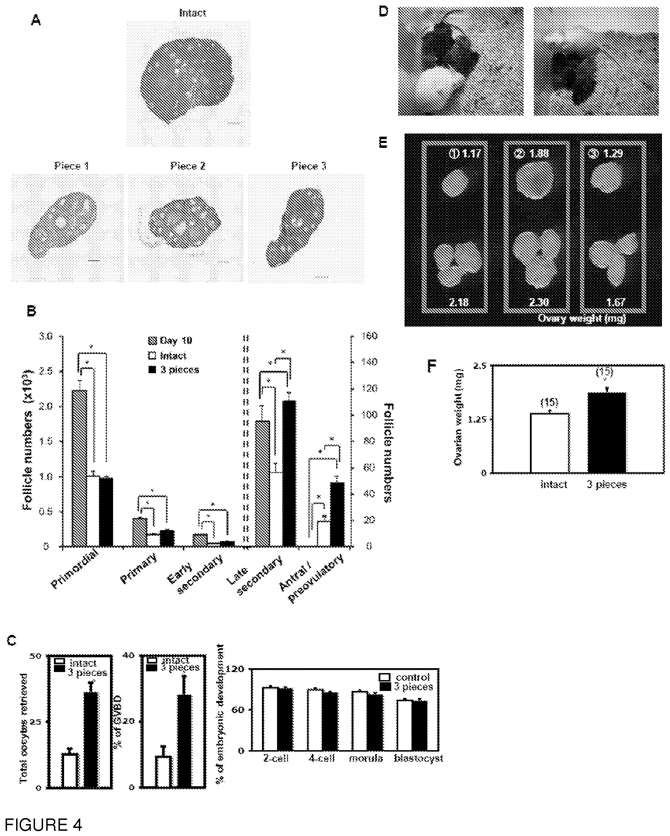

Fig. 4 Ovarian fragmentation/grafting led to viable pups. A, Representative histology of ovarian grafts from day 10 mice with or without cutting into three pieces, followed by grafting for 5 days. Bars=200µm. B, Absolute follicle numbers before and after grafting of intact and fragmented (3 pieces) ovaries from mice at day 10 of age. Left: total follicle numbers, Right: follicle dynamics. n=5 ovaries. Same asterisk symbols indicate significant differences (p<0.05). C, Retrieval of oocytes (left panel) after cutting/grafting of ovaries from day 10 mice for fertilization and embryonic development. Percentage of mature oocytes (middle panel) and fractions of fertilized oocytes developed to different embryonic stages (right panel) are shown. Controls in the right panel represent oocytes obtained after treatment of day 23 mice with eCG, followed by hCG to induce ovulation(3). n=8 ovaries. GVBD: germinal vesicle breakdown. D. Delivery of healthy pups following transfer of embryos derived from mature oocytes obtained after ovarian fragmentation/grafting. Ovaries from day 10 B6D2F1 mice were fragmented into three pieces before grafting for 5 days with daily FSH treatment. Animals were then treated sequentially with eCG (48h) and hCG (12h) before retrieval of mature oocytes for fertilization and embryo culture. Two-cell embryos were transferred to surrogate mothers of the CD1 strain followed by pregnancy and delivery. Pups derived from ovarian fragmentation were healthy and fertile. E and F, Ovarian fragmentation and grafting promoted ovarian follicle development in rats. Paired ovaries from day 10 rats were auto-grafted into kidneys of the same animals intact or in three pieces. Animals were treated with FSH for five days before determination of ovarian weights. E, Morphology of isolated ovaries. F. Ovarian weights. Numbers in parentheses are number of ovaries used. Mean+/-SEM, p<0.05.

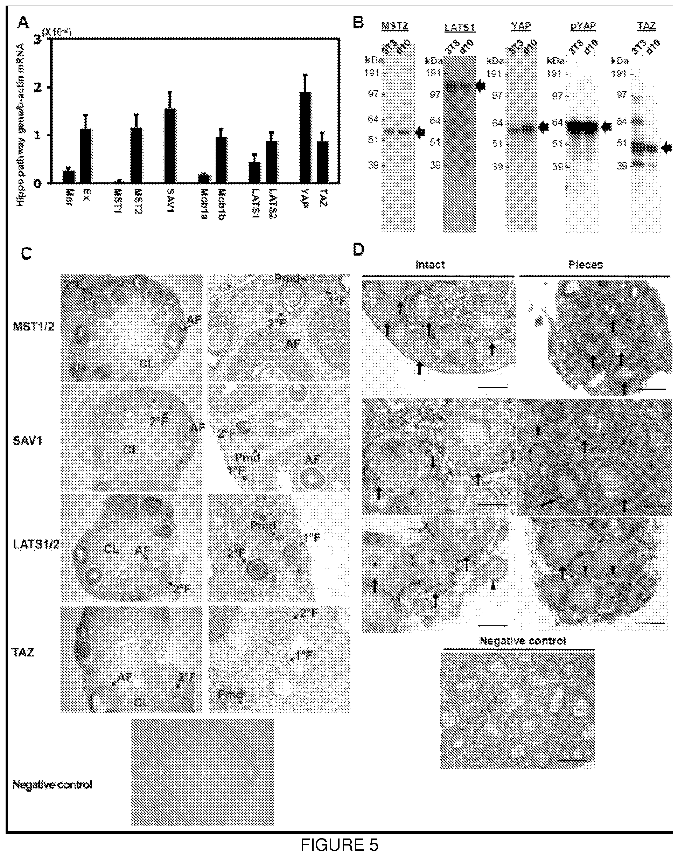

Fig. 5. Ovarian expression of Hippo pathway genes and increases in nuclear YAP localization following ovarian fragmentation. A, Real-time RT-PCR analyses of transcripts for Hippo pathway genes in ovaries of day 10 mice. n=6. B, Immunoblotting of Hippo pathway proteins in ovaries of mice at day 10 of age (d10). Extracts from 3T3 cells served as controls (3T3). Specific signals are marked with arrows. pYAP: phospho-YAP(Ser127). C, Immunohistochemical staining of Hippo signaling genes in ovaries of adult mice. Signals were detected using specific antibodies. All antigens were found mainly in the cytoplasm of granulosa cells, theca cells, and oocytes of primordial (Pmd), primary (1°F), secondary (2°F), and antral (AF) follicles but at lower levels in the corpus luteum (CL). Scale bars, 200 and 100 µm for low (left panel) and high (right panel) magnification, respectively. D, Fragmentation-induced increases in nuclear YAP in granulosa cells of primary and secondary follicles. Left panels: YAP staining in intact ovaries from mice at day 10 of age, showing predominantly cytoplasmic localization of YAP in granulosa cells of most primary and secondary follicles. Right panels: YAP staining in ovaries at 4h after fragmentation showing nuclear localization of YAP in granulosa cells of most primary and secondary follicles. Due to limited cytoplasm of granulosa cells in primordial follicles, it is difficult to determine cellular distribution of YAP in primordial follicles. Scale bars, 100 and 50 µm for low (upper panels) and high (middle and lower panels) magnification, respectively. Arrows; secondary follicles, arrowheads; primary follicles.

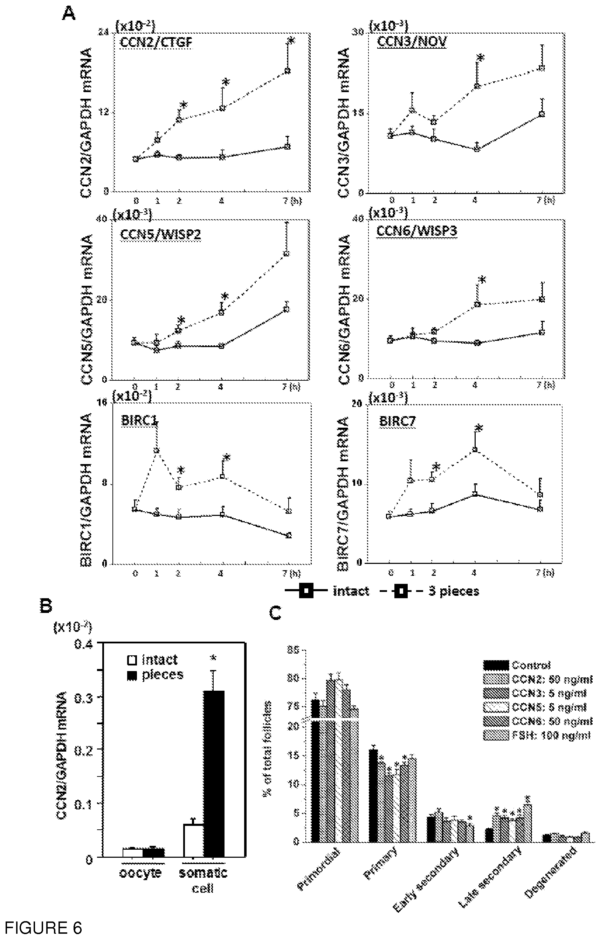

Fig. 6. Increased expression of CCN growth factors and BIRC inhibitors after ovarian fragmentation and the ability of CCN growth factors to promote follicle development. A, Ovarian fragmentation without subsequent grafting increased the expression of key CCN growth factors (CCN2, 3, 5, and 6) and apoptosis inhibitors (BIRC1 and 7). Paired ovaries from day 10 mice with or without cutting into 3 pieces were incubated for up to 7h before analyses of transcript levels for different genes. Intact ovaries: solid lines; three pieces: dashed lines. n=10-15. B, Increases in CCN2 expression in somatic cells of fragmented ovaries. Paired ovaries from day 10 mice with or without cutting into 3 pieces were cultured for 1 h and then transplanted under kidney capsules. At 3 h after transplantation, ovaries were dissected followed by isolation of oocytes and somatic cells as previously described (2). CCN2 and GAPDH mRNA levels were measured by real-time RT-PCR (n=4). *, P < 0.05 vs intact. C. Treatment with CCN growth factors promoted preantral follicle development in ovarian explants. Following explant cultures with different CCN growth factors, follicle dynamics were determined by counting the percentages of follicles at different developmental stages. Mean±SEM; *p<0.05, n=5.

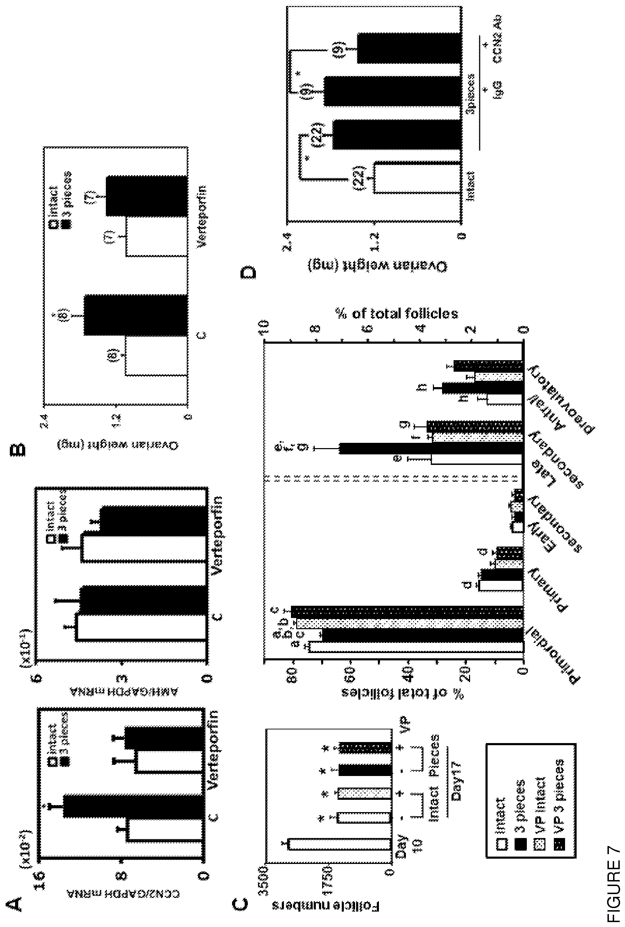

Fig. 7. Pre-treatment with verteporfin and CCN2 antibodies suppressed fragmentation-induced ovarian growth. A, Pretreatment with verteporfin suppressed fragmentation-induced increases in CCN2, but not AMH, transcript levels. Juvenile mice were injected i.p. with verteporfin for 3h before retrieving ovaries for fragmentation. At 4h after incubation, transcript levels for CCN2 and AMH were determined by real-time RT-PCR. n=6-12. B, Pretreatment with verteporfin partially suppressed fragmentation-induced graft weight increases. Ovaries from juvenile mice pre-injected with verteporfin for 3h were fragmented and incubated for 1h before grafting into FSH-treated hosts for 5 days followed by determination of graft weights. Numbers in parentheses are number of samples used. C, Follicle dynamics of intact and fragmented ovarian grafts from animals with or without verteporfin (VP) pretreatment. n=3-5. D, Incubation of fragmented ovaries with CCN2 antibodies suppressed ovarian weight increases. Paired ovaries were fragmented into 3 pieces followed by incubation with CCN2 antibodies or non-immune-IgG for 18h before grafting for 5 days. Mean±SEM; *p<0.05.

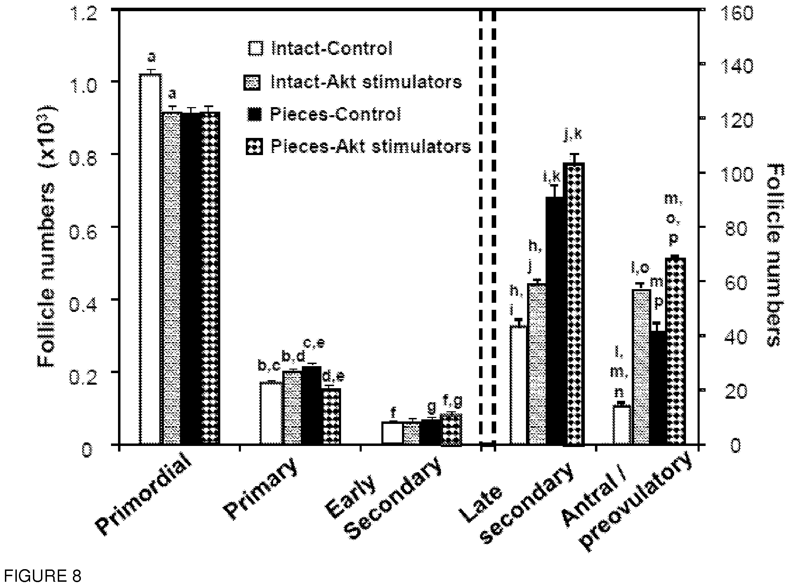

Fig. 8. Absolute follicle numbers after grafting of intact and fragmented ovaries with or without treatment with Akt stimulators. Paired ovaries from juvenile mice were fragmented and incubated with or without Akt stimulators for 2 days followed by allo-transplantation for 5 days to determine graft weights. n =4. Same letter symbols indicate significant differences (p<0.05).

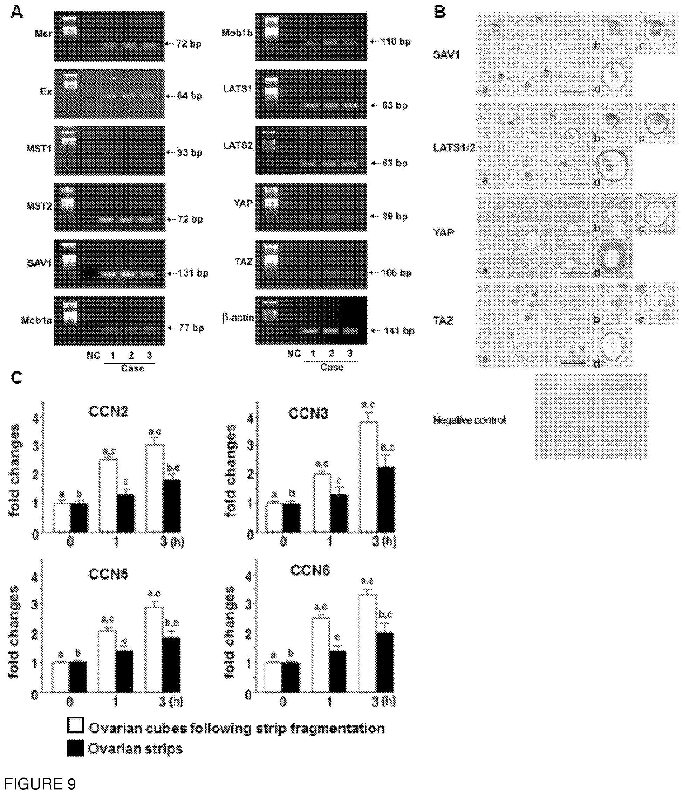

Fig. 9. Expression of Hippo signaling genes in human ovaries. A, Expression of transcripts for Hippo signaling genes in human ovarian cortices. Ovarian cortical tissues were obtained from patients with benign ovarian tumor and used for RT-PCR analyses. B, Immunohisto-chemical staining of Hippo signaling proteins in human ovarian cortices. Expression of SAV1, LATS1/2, YAP, and TAZ antigens in ovarian cortices was performed. All antigens were found in granulosa cells, theca cells, and oocytes of primordial (b), primary (c), and secondary (d) follicles. Scale bars, 100 µm. C, Increases in CCN transcripts after fragmentation of human ovarian tissues. Thawed cortical strips were cut into pieces or left intact before incubation for different intervals followed by real-time RT-PCR analyses. n=4-8.

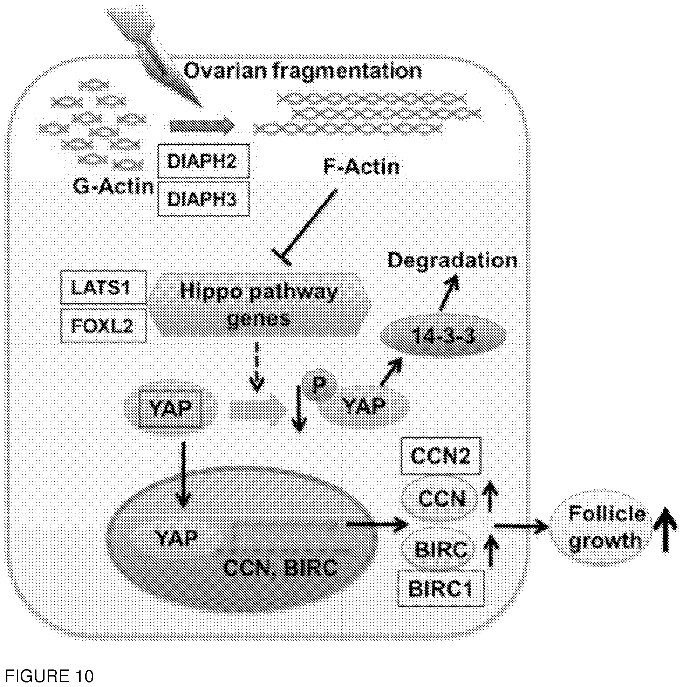

Fig. 10. Genes involved in ovarian fragmentation, Hippo signaling, and follicle growth are important for ovarian physiology and pathophysiology. Ovarian fragmentation led to changes in intercellular tension and facilitated the conversion of G-actin to F-actin. Subsequent disruption of Hippo signaling decreased pYAP to total YAP ratios, leading to increased expression of downstream CCN growth factors and BIRC apoptosis inhibitors. Secretion of CCN growth factors stimulated follicle growth. Genetic studies found DIPAH2 and FOXL2 mutations in POI families whereas deletion of LATS1 or CCN2 led to infertility phenotypes in mice. Genome-wide association studies identified DIAPH2 and DAIPH3 as candidate genes for follicle reserve and menopausal ages whereas copy number changes for BIRC1 were found in POI patients. YAP was found not only as a candidate gene for PCOS but also as an oncogene for ovarian surface epithelial cancer.

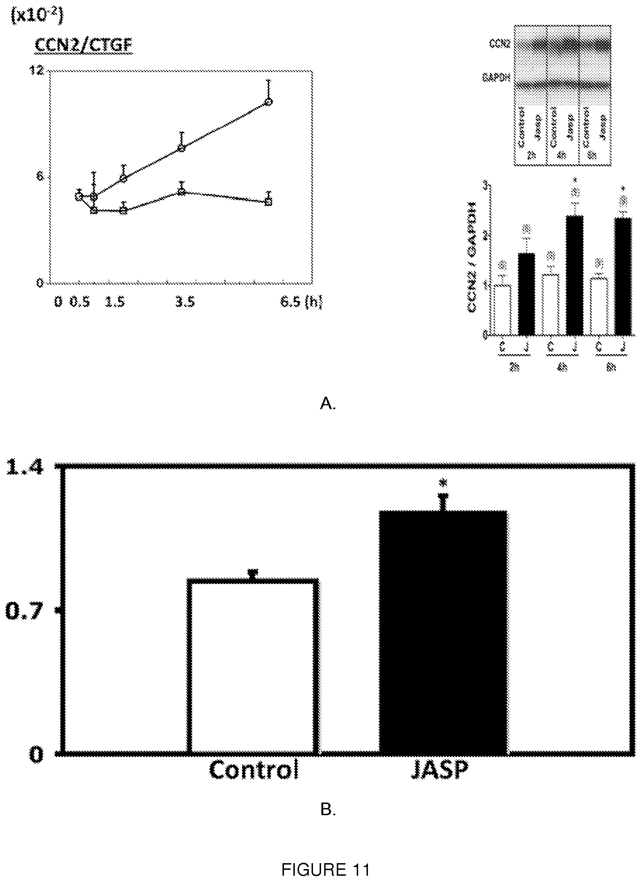

Fig. 11A-B. Treatment with JASP increased actin polymerization and CCN2 expression. Left upper panel: Paired ovaries from day 10 mice were treated with or without JASP (10 µM) for 30 min. before analyses of F- and G-actin content. Left lower panel: Real-time RT-PCR analyses of CCN2 transcript levels after 30 min. exposure to JASP. Right panel: Immunoblotting analyses of CCN2 protein levels after 30 min. treatment with JASP. Upper panel: Immunoblotting analyses. Lower panel: quantitative analyses of densitometric scanning data. Numbers in parentheses are number of samples used. * p<0.05. C) Exposure of ovaries to JASP increased weights of ovarian grafts. Ovaries from day 10 mice were treated with or without different concentrations of JASP for 30 min. before grafting into adult hosts treated daily with FSH. Five days later, graft weights were determined.

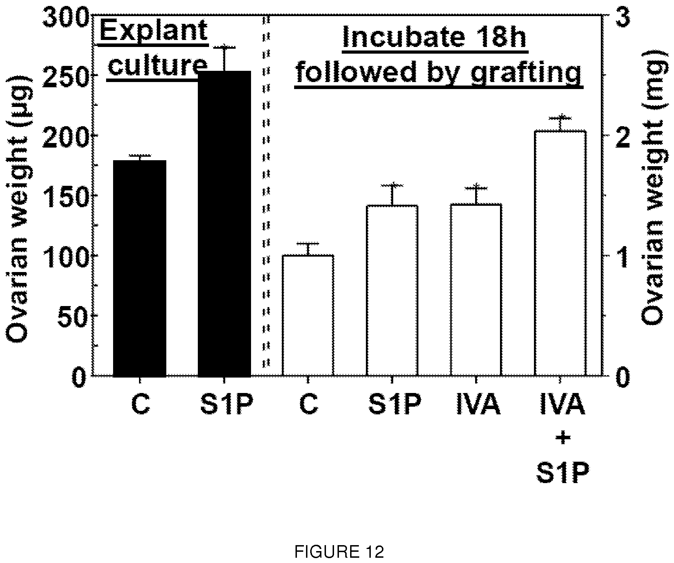

Figure 12. Addition of S1P to explant cultures of ovaries leads to major increases in ovarian weights. Culturing day 10 ovaries with S1P (6 µM) for 18h, followed by grafting into adult ovariectomized hosts for 5 days also led to increases in graft weights (right panel). When ovaries are treated with S1P plus the IVA drugs (PTEN inhibitors and PI3K stimulator), additive increases in graft weights are found.

### DESCRIPTION OF THE SPECIFIC EMBODIMENTS

Compositions and methods are provided for modulating the growth and maturation of mammalian ovarian follicles. By exposing follicles to an effective dose of at least one of an agent that disrupts signaling in the Hippo pathway, or an agent that acts downstream of disrupted Hippo signaling, follicle growth and consequent oocyte maturation can be manipulated.

The methods of the disclosure find use in a wide variety of animal species, particularly including mammalian species. Animal models, particularly small mammals, e.g. murine, lagomorpha, *etc.* are of interest for experimental investigations. Other animal species may benefit from improvements in *in vitro* fertilization, *e.g.* horses, cattle, rare zoo animals such as panda bears, large cats, *etc.* Humans are of particular interest for enhancing oocyte maturation, including methods of *in vitro* fertilization. Individuals of interest for treatment with the methods of the disclosure include, without limitation, those suffering from premature ovarian failure, peri-menopause, FSH low responsiveness, polycystic ovarian syndrome, age-related infertility, i.e. woman greater tha 40 years of age, etc.

Disclosure herein can include ovarian follicles of numerous species of mammals. The disclosure should be understood not to be limited to the species of mammals cited by the specific examples within this patent application. Disclosure herein, for example, may include fresh or frozen-thawed follicles of animals having commercial value for meat or dairy production such as swine, bovids, ovids, equids, buffalo, or the like (naturally the mammals used for meat or dairy production may vary from culture to culture). It may also include ovarian follicles from individuals having rare or uncommon attribute(s), such as morphological characteristics including weight, size, or conformation, or other desired characteristics such as speed, agility, intellect, or the like. It may include ovarian follicles from deceased donors, or from rare or exotic mammals, such as zoological specimens or endangered species. Disclosure herein may also include fresh or frozen-thawed ovarian follicles collected from primates, including but not limited to, chimpanzees, gorillas, or the like, and may also ovarian follicles from marine mammals, such as whales or porpoises.

Before the subject invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs.

*Ovarian follicle.* An ovarian follicle is the basic unit of female reproductive biology and is composed of roughly spherical aggregations of cells found in the ovary. A follicle contains a single oocyte. Follicles are periodically initiated to grow and develop, culminating in ovulation of usually a single competent oocyte. The cells of the ovarian follicle are the oocyte, granulosa cells and the cells of the internal and external theca layers. The oocyte in a follicle is in the stage of a primary oocyte. The nucleus of such an oocyte is called a germinal vesicle. Granulosa cells within the follicle surround the oocyte; their numbers increase in response to gonadotropins. They also produce peptides involved in ovarian hormone synthesis regulation. Follicle-stimulating hormone (FSH) acts on granulosa cells to express luteinizing hormone (LH) receptors on the cell surface. The granulosa cells, in turn, are enclosed in a thin layer of extracellular matrix - the follicular basement membrane or basal lamina. Outside the basal lamina, the layers theca interna and theca externa are found.

*Ovarian in vitro culture.* Methods are known in the art for culturing mammalian ovaries or fragments thereof, which fragments for the purposes of the present disclosure will include at least one follicle. Typically all or a portion of an ovary is placed in tissue culture medium, which medium may include factors useful in the growth or maintenance of the follicle cells, and which, as described herein, further comprise an effective dose of at least one of an agent that disrupts signaling in the Hippo pathway, or an agent that acts downstream of disrupted Hippo signaling. See the Examples provided herein. Additional description may be found, *inter alia,* at Hoyer et al. (2007) Birth Defects Res B Dev Reprod Toxicol. 80(2):113-25. In vitro culture of canine ovaries is described by Luvoni et al. (2005) Theriogenology.;63(1):41-59. Culture of bovine follicles is described by Hansel (2003) Anim Reprod Sci.;79(3-4):191-201.

A review of in vitro ovarian tissue and organ culture may be found in Devine et al. (2002) Front Biosci. 7:d1979-89; and in Smitz et al. (2002) Reproduction. 123(2):185-202. Whole ovaries from fetal or neonatal rodents have been incubated in organ culture systems. Adaptations of this technique include incubation of ovaries in a chamber continuously perfused with medium or perfusion of medium through the intact vasculature. Another approach has been to culture individual follicles isolated by enzymatic or mechanical dissociation. Cryopreservation of human primordial and primary ovarian follicles is described by Hovatta (2000) Mol Cell Endocrinol. 169(1-2):95-7.

*Ovarian transplantation.* Ovarian transplantation to the kidney is a well-established procedure in animal studies. Autologous transplantation of ovarian cortical tissue has been widely reported in humans, particularly in the context of women undergoing sterilizing cancer therapy or surgery. Ovarian tissue may be transplanted fresh, or after cryo-preservation. For a review, see Grynberg et al. (2012) Fertil. Steril. 97(6):1260-8.

*Agents that disrupt Hippo signaling.* Agents that increase actin polymerization disrupt Hippo signaling. A number of such agents are known in the art, including phalloidins, which bind to actin filaments at a ratio of one molecule for either one or two actin protomers and essentially lock adjacent actin subunits together. This shifts the equilibrium to favour filament formation over filament disassembly. See, for example, Cooper (1987) J. Cell Biol. 105(4):1473-8.

Jasplakinolide is a 15-carbon macrocyclic peptide that has been shown to have a number of effects on the actin cytoskeleton. These include inducing spontaneous nucleation of G-actin monomers and actin polymerization. Like phalloidin, jasplakinolide stabilizes F-actin filaments by inihibiting filament disassembly. Whilst the two drugs have substantially different structures, jasplakinolide has been shown to bind competitively with phalloidin to F-actin, suggesting a similar binding mechanism, and possibly a similar mode of action. See, for example, Bubb et al. (1994) J. Biol. Chem. 269(21):14869-71. The effective concentration of JASP for in vitro culture may be from about 0.1 µM, about 1 µM, about 10 µM, about 50 µM, and not more than about 1 mM. For *in vivo* purposes the dose may vary depending on the individual and the manner of dosing, e.g. it may be desirable to localize the agent so as to achieve a higher concentration in the targeted tissue.

*Sphingosine 1-phosphate receptor modulator.* Included among agents that disrupt Hippo signaling are sphingosine 1-phosphate receptor modulators. Sphingosine 1-phosphate (S1P) is a bioactive sphingolipid, acting both as an intracellular second messenger and extracellular mediator, in mammalian cells. In cell types where S1P acts as an intracellular messenger, stimulation-dependent synthesis of S1P, resulting from sphingosine kinase activation, is essential.

Extracellular S1P acts through a family of five related G protein-coupled receptors, S1PR1-5 (see Rosen et al. (2009). Ann. Rev. Biochem. 78:743-768). These S1P receptors are differentially expressed, coupled to various G proteins, and regulate angiogenesis, vascular maturation, cardiac development and immunity (Spiegel & Milstien (2003) Nature Reviews Molecular Cell Biology 4, 397-407). S1P acts through G12/13-coupled S1P receptors to increase actin polymerization and to inhibit the kinases Lats1/2, thereby activating YAP and TAZ transcription coactivators and promoting cell proliferation.

S1P is abundantly stored in platelets and is a normal constituent of human serum and follicular fluid (Becker et al. (2011) Biology of Reproduction 84, 604-612). Of interest, granulosa cells express four of the five S1P receptors and follicular fluid high-density lipoprotein-associated S1P promotes actin polymerization and migration of human granulosa lutein cells.

Agents of interest for this purpose include, without limitation, S1P, Fingolimod (FTY720, trade name Gilenya, Novartis), and lysophosphatidic acid (LPA). Fingolimod is a sphingosine 1-phosphate receptor modulator, which sequesters lymphocytes in lymph nodes, preventing them from contributing to an autoimmune reaction. First synthesized in 1992 by Yoshitomi Pharmaceuticals, it was derived from an immuno-suppressive natural product, myriocin through chemical modification.

The effective concentration of S1P, FTY720, LPA for in vitro culture may be from about 0.1 µM, from about 1 µM, from about 5 µM, up to about 50 µM, and not more than about 1 mM. For *in vivo* purposes the dose may vary depending on the individual and the manner of dosing, e.g. it may be desirable to localize the agent so as to achieve a higher concentration in the targeted tissue. In some disclosure herein the agent is directly injected into an ovary, e.g. at a dose of from about 0.01 mg/kg, from about 0.05 mg/kg, from about 0.1 mg/kg, from about 0.5 mg/kg, from about 1 mg/kg, from about 5 mg/kg, up to about 100 mg/kg, up to about 50 mg/kg, up to about 10 mg/kg.

*Agents that act downstream of disrupted Hippo signaling* include CCN proteins, particularly CCN 2, 3, 5 and 6. CCN intercellular signaling protein refers to the CCN family of extracellular matrix (ECM)-associated signaling proteins (also known as matricellular proteins). Members of the CCN protein family are characterized by having four conserved cysteine-rich domains, which include the insulin-like growth factor-binding domain (IGFBP), the Von Willebrand factor type C domain (VWC), the thrombospondin type 1 repeat (TSR), and a C-terminal domain (CT) with a cysteine knot motif. Collectively, these proteins stimulate mitosis, adhesion, apoptosis, extracellular matrix (ECM) production, growth arrest and migration, and regulate angiogenesis, tumor growth, placentation, implantation, embryogenesis and endochondral ossification. CCN proteins block apoptosis.

CCN2 is also known as CTGF (connective tissue growth factor); CCN3 is also known as NOV (nephroblastoma overexpressed); CCN5 is also known as WISP2 (WNT1 inducible signaling pathway protein-2); and CCN6 is also known as WISP3 (WNT1 inducible signaling pathway protein-3).

For the purposes of the disclosure, CCN proteins may be isolated from natural sources, or may be produced recombinantly. The proteins are optionally purified, e.g. by column chromatography, affinity chromatography, and the like as known in the art. Generally the protein will be derived from same species as the follicles, i.e. human proteins for human follicles, etc., although in some instances there may be cross-reactivity that allows the protein from one species to work with another.

For *in vitro* culture, the effective dose of a CCN protein is usually at least about 1 ng/ml, at least about 10 ng/ml, at least about 50 ng/ml, at least about 100 ng/ml, and not more than about 100 µg/ml. For *in vivo* purposes the dose may vary depending on the individual and the manner of dosing, e.g. it may be desirable to localize the protein so as to achieve a higher concentration in the targeted tissue.

FSH. Follicle-stimulating hormone (FSH) is a hormone synthesized and secreted by gonadotropes in the anterior pituitary gland. FSH regulates the development, growth, pubertal maturation, and reproductive processes of the human body. FSH and Luteinizing hormone (LH) act synergistically in reproduction. In females, in the ovary FSH stimulates the growth of immature follicles to maturation. As the follicle grows, it releases inhibin, which shuts off the FSH production.

FSH is a dimeric glycoprotein. The alpha subunits of LH, FSH, TSH, and hCG are identical, and contain 92 amino acids. FSH has a beta subunit of 118 amino acids (FSHB), which confers its specific biologic action and is responsible for interaction with the FSH-receptor. The half-life of native FSH is 3-4 hours. Its molecular wt is 30000.

Various formulations of FSH are available for clinical use. It is used commonly in infertility therapy to stimulate follicular development, notably in IVF therapy, as well as with interuterine insemination (IUI). FSH is available mixed with LH in the form of Pergonal or Menopur, and other more purified forms of urinary gonadotropins, as well as in a pure forms as recombinant FSH (Gonal F, Follistim), and as Follistim AQ, Gonal-F, Gonal-f RFF, Gonal-f RFF Pen.

Analogs of FSH are also clinically useful, which analogs include all biologically active mutant forms, e.g. where one, two, three or more amino acids are altered from the native form, PEGylated FSH, single chain bi-functional mutants, FSH-CTP, and the like. In an effort to enhance ovarian response several long-acting FSH therapies have been developed including an FSH-CTP (Corifollitropin alfa), where the FSH beta subunits are linked by the C-terminal peptide (CTP) moiety from human chorionic gonadotropin (hCG); and single-chain bi-functional VEGF-FSH-CTP (VFC) analog. FSH-CTP has a longer half-life in vivo, and may be administered, for example, with an interval of from one to four weeks between doses. See, for example, Lapolt et al. (1992) Endocrinology 131:2514-2520; and Devroey et al. (2004) The Journal of Clinical Endocrinology & Metabolism Vol. 89, No. 5 2062-2070.

*LH and agonists.* LH is a heterodimeric glycoprotein. Its structure is similar to that of the other glycoprotein hormones, follicle-stimulating hormone (FSH), thyroid-stimulating hormone (TSH), and human chorionic gonadotropin (hCG). The protein dimer contains 2 glycopeptidic subunits, labeled alpha and beta subunits, that are non-covalently associated. The alpha subunits of LH, FSH, TSH, and hCG are identical, and contain 92 amino acids in human but 96 amino acids in almost all other vertebrate species. The beta subunits vary. LH has a beta subunit of 120 amino acids (LHB) that confers its specific biologic action and is responsible for the specificity of the interaction with the LH receptor. This beta subunit if highly homologous to the beta subunit of hCG and both stimulate the same receptor. LH is available mixed with FSH in the form of Pergonal, and other forms of urinary gonadotropins Recombinant LH is available as lutropin alfa (Luveris). All these medications are administered parenterally.

Often, hCG medication is used as an LH substitute because it activates the same receptor, is less costly, and has a longer half-life than LH. Human chorionic gonadotropin is a glycoprotein of 244 amino acids. The β-subunit of hCG gonadotropin contains 145 amino acids. Like other gonadotropins, hCG can be extracted from urine or by genetic modification. Pregnyl, Follutein, Profasi, Choragon and Novarel use the former method, derived from the urine of pregnant women. Ovidrel is a product of recombinant DNA. As an alternative, equine chorionic gonadotropin (eCG) is a gonadotropic hormone produced in the chorion of pregnant mares.

*PTEN inhibitor.* The polypeptide PTEN (phosphatase with TENsin homology) was identified as a tumor suppressor that is mutated in a large number of cancers at high frequency. The protein encoded this gene is a phosphatidylinositol-3,4,5-trisphosphate 3-phosphatase. It contains a tensin like domain as well as a catalytic domain similar to that of the dual specificity protein tyrosine phosphatases. Unlike most of the protein tyrosine phosphatases, this protein preferentially dephosphorylates phosphoinositide substrates. It negatively regulates intracellular levels of phosphatidylinositol-3,4,5-trisphosphate in cells and functions as a tumor suppressor by negatively regulating AKT/PKB signaling pathway. The genetic sequence of the human protein may be found in Genbank, accession number NM_000314, as described by Volinia et al. (2008) PLoS ONE 3 (10), E3380; Li et al. (1997) Cancer Res. 57 (11), 2124-2129; Steck et al. (1997) Nat. Genet. 15 (4), 356-362; and Li et al. (1997) Science 275 (5308), 1943-1947. PTEN inhibitors of interest may have an IC₅₀ of from about 0.1 nM to about 100 µM, and may be from about 1 nm to about 10 µM, of from about 10 nM to about 1 µM, of from about 1 nM to about 100 nM.

A number of known PTEN inhibitors are known in the art, including without limitation, bisperoxovanadium compounds (see, for example, Schmid *et al.* (2004) FEBS Lett. 566(1-3):35-8). Included are potassium bisperoxo(bipyridine)oxovanadate (V), which inhibits PTEN at an IC₅₀ = 18 nM; dipotassium bisperoxo(5-hydroxypyridine-2-carboxyl)oxovanadate (V), which inhibits PTEN at an IC₅₀ = 14 nM; potassium bisperoxo (1,10-phenanthroline)oxovanadate (V) which inhibits PTEN at an IC₅₀ = 38 nM; dipotassium bisperoxo(picolinato)oxovanadate (V) which inhibits PTEN at an IC₅₀ = 31 nM; N-(2-Hydroxy-3-methoxy-5-dimethylamino)benzyl, N'-(2-(4-nitrophenethyl)), N"-methylamine which inhibits the CDC25 phosphatase family; dephostatin which is a competitive PTP inhibitor; monoperoxo(picolinato)oxovanadate(V) which is a PTP inhibitor(IC₅₀ = 18 µM); and sodium orthovanadate, which is a broad-spectrum inhibitor of phosphatases.

Additional PTEN inhibitors are described by, *inter alia,* Myers et al. (1998) PNAS 95:13513-13518; by Garlich et al., WO/2005/097119; and by Rosivatz et al. (2007) ACS Chem. Biol., 1, 780-790.

Alternatively, inhibitors of PTEN may be identified by compound screening for agents, *e.g.* polynucleotides, antibodies, small molecules, *etc.,* that inhibit the enzymatic activity of PTEN, which is known to have phosphatase activity. Compound screening may be performed using an in vitro model, a genetically altered cell or animal or purified PTEN1 protein. One can identify ligands or substrates that bind to or inhibit the phosphatase activity. A wide variety of assays may be used for this purpose, including labeled in vitro protein-protein binding assays, electrophoretic mobility shift assays, immunoassays for protein binding, and the like. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. A variety of other reagents may be included in the screening assay. These include reagents like salts, neutral proteins, e.g. albumin, detergents, etc. that are used to facilitate optimal protein-protein binding and/or reduce non-specific or background interactions. Reagents that improve the efficiency of the assay, such as protease inhibitors, nuclease inhibitors, anti-microbial agents, etc. may be used. The mixture of components are added in any order that provides for the requisite binding. Incubations are performed at any suitable temperature, typically between 4 and 40° C. Incubation periods are selected for optimum activity, but may also be optimized to facilitate rapid high-throughput screening. Typically between 0.1 and 1 hours will be sufficient.

PI3K activator. Phosphoinositide 3-kinases (PI 3-kinases or PI3Ks) are a family of enzymes involved in cellular functions such as cell growth, proliferation, differentiation, motility, survival and intracellular trafficking, which are capable of phosphorylating the 3 position hydroxyl group of the inositol ring of phosphatidylinositol (Ptdlns).

Class I PI3Ks are responsible for the production of Phosphatidylinositol 3-phosphate (PI(3)P), Phosphatidylinositol (3,4)-bisphosphate (PI(3,4)P2) and Phosphatidylinositol (3,4,5)-trisphosphate (PI(3,4,5)P₃. The PI3K is activated by G-protein coupled receptors and tyrosine kinase receptors.

Class I PI3K are heterodimeric molecules composed of a regulatory and a catalytic subunit; which are further divided between IA and IB subsets on sequence similarity. Class I PI 3-kinases are composed of a catalytic subunit known as p110 and a regulatory subunit either related to p85 or p101. The p85 subunits contain SH2 and SH3 domains.

Activators of PI3K increase the activity of the enzyme. Activators of interest include, without limitation the cell-permeable phospho-peptide (740Y-P), which is capable of binding to the SH2 domain of the p85 regulatory subunit of PI3K to stimulate enzyme activity (commercially available peptide, RQIKIWFQNRRMKWKKSDGGYMDMS, Modifications: Tyr-25 = pTyr). Other activators include fMLP (see Inoue T, Meyer T (2008) Synthetic Activation of Endogenous PI3K and Rac Identifies an AND-Gate Switch for Cell Polarization and Migration. PLoS ONE 3(8): e3068. Also see Bastian et al., Mol Cancer Res 2006;4(6). June 2006; Park et al. Toxicology Toxicology Volume 265, Issue 3, 30 November 2009, Pages 80-86)

*Candidates for Therapy.* Any female human subject who possesses viable ovarian follicles is a candidate for therapy with the methods of the disclosure. Typically, the subject will suffer from some form of infertility, including premature ovarian failure. For instance, the subject may experience normal oocyte production but have an impediment to fertilization, as in, e.g. PCOS or PCOS-like ovaries. The methods of the disclosure may be especially useful in women who are not suitable candidates for traditional *in vitro* fertilization techniques involving an ovarian stimulation protocol. Included are patients with low responses to the conventional FSH treatment.

As described above, the methods of the disclosure are also useful in the treatment of infertility with various non-human animals, usually mammals, e.g. equines, canines, bovines, *etc.*

Premature ovarian failure (POF) occurs in 1% of women. The known causes for POF include genetic aberrations involving the X chromosome or autosomes as well as autoimmune ovarian damages. Presently, the only proven means for infertility treatment in POF patients involve assisted conception with donated oocytes. Although embryo cryopreservation, ovarian cryopreservation, and oocyte cryopreservation hold promise in cases where ovarian failure is foreseeable as in women undergoing cancer treatments, there are few other options. Due to heterogeneity of POF etiology, varying amounts of residual primordial follicle are still present in patients' ovaries for activation.

The degrees of ovarian follicle exhaustion vary among POF patients. The methods of the present disclosure allow the activation of the remaining follicles in POF patients using the methods of the disclosure to promote the development of early follicles to the preovulatory stage. This may be followed by the retrieval of mature oocytes for IVF and subsequent pregnancy following embryo transfer.

Due to the delay of child-bearing age in the modern society, many women also are experiencing infertility as the result of diminishing ovarian reserve during aging, e.g. infertile women of from about 40-45 years of age. Although gonadotropin treatments are widely used to promote the development of early antral follicles to the preovulatory stage, many perimenopausal patients do not respond to the gonadotropin therapy. Because these women still have varying numbers of primordial follicles, they also benefit from the methods of the disclosure.

*Polycystic ovary syndrome* is a clinical syndrome characterized by mild obesity, irregular menses or amenorrhea, and signs of androgen excess (eg, hirsutism, acne). In most patients, the ovaries contain multiple cysts. Diagnosis is by pregnancy testing, hormone measurement, and imaging to exclude a virilizing tumor. Treatment is symptomatic. Polycystic ovary syndrome occurs in 5 to 10% of women and involves anovulation or ovulatory dysfunction and androgen excess of unclear etiology. It is usually defined as a clinical syndrome, not by the presence of ovarian cysts. Ovaries may be enlarged with smooth, thickened capsules or may be normal in size. Typically, ovaries contain many 2- to 6-mm follicular cysts and sometimes larger cysts containing atretic cells. Estrogen levels are elevated, increasing risk of endometrial hyperplasia and, eventually, endometrial cancer. Androgen levels are often elevated, increasing risk of metabolic syndrome and causing hirsutism. Over the long term, androgen excess increases risk of cardiovascular disorders, including hypertension.

### METHODS OF ENHANCING OOOCYTE MATURATION

Methods are provided for promoting the development of mammalian ovarian follicles *in vitro* and *in vivo,* by contacting follicles with an effective dose of at least one of an agent that disrupts signaling in the Hippo pathway, or an agent that acts downstream of disrupted Hippo signaling, for a period of time sufficient to stimulate the development to antral and preovulatory follicle.

Optionally, one or both of an inhibitor of PTEN and an activator of PI3K are also brought into contact with the follicle, at a concentration that is effective to additively induce the follicles to initiate growth.

In some disclosure herein, the exposure is performed *in vitro, e.g.* in an organ or tissue culture, where at least one ovarian follicle is transiently exposed to an effective dose of at least one of an agent that disrupts signaling in the Hippo pathway, or an agent that acts downstream of disrupted Hippo signaling. In some disclosure herein an intact ovary is thus treated.

The treated follicle may be utilized for *in vitro* purposes, for example for *in vitro* fertilization, generation of embryonic stem cells, *etc.,* or may be transplanted to provide for *in vivo* uses. Transplantation modes of interest include, without limitation, transplantation of one or more follicles, including all or a fraction of an ovary, to a kidney capsule, to Fallpian tubes, to a subcutaneous site, to an ovarian site, e.g. where one ovary has been retained and one has been removed for *ex vivo* treatment, the one or more treated follicles may be transplated to the site of the remaining ovary.

In some disclosure herein, an *in vitro method* combines treatment with Hippo pathway disruption, by cutting an ovary to disrupt Hippo signaling or by contacting at least one follicle with at least one of an agent that disrupts signaling in the Hippo pathway, or an agent that acts downstream of disrupted Hippo signaling; and further contacting the ovarian follicles with at least one of a phosphatase and tensin homolog (PTEN) inhibitor, and a phosphatidylinositol 3-kinase (PI3 kinase) activator, which provides for an additive effect to stimulate growth and differentiation of the follicle.

In some disclosure herein, *in vitro* treatment is followed by ovarian transplantation, which may be followed by *in vitro* or *in vivo* fertilization.

Following exposure to an effective dose of at least one of an agent that disrupts signaling in the Hippo pathway, or an agent that acts downstream of disrupted Hippo signaling, the individual may be treated with follicular stimulating hormone (FSH) or FSH analogs, including recombinant FSH, naturally occurring FSH in an *in vivo* host animal, FSH analogs, e.g. FSH-CTP, pegylated FSH, and the like, at a concentration that is effective to initiate follicular growth.

Where the follicles have been stimulated to the antral stage, the individual may be treated lutenizing hormone (LH) or an agonist thereof, which agonists specifically include chorionic gonadotropins, e.g. equine chorionic gonadotropin (eCG), human chorionic gonatotropin (HCG), *etc.,* at an ovulatory dose. In addition, the follicles may be exposed *in vivo* or *in vitro* to one or more of c-kit ligand, neurotrophins, vascular endothelial growth factor (VEGF), bone morphogenetic protein (BMP)-4, BMP7, leukemia inhibitory factor, basic FGF, keratinocyte growth factor; and the like.

The dose of an agent that disrupts signaling in the Hippo pathway, or an agent that acts downstream of disrupted Hippo signaling is sufficient to stimulate pre-antral follicles to induce antral development as described above, and as such, will vary according to the specific agent that is used, the length of time it is provided in the culture, the condition of the follicles, etc. Methods known in the art for empirical determination of concentration may be used. Toxicity and therapeutic efficacy of the active ingredient can be determined according to standard pharmaceutical procedures in cell cultures and/or experimental animals, including, for example, determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred.

As an example, follicle cultures may be contacted with one or both of an agent that disrupts signaling in the Hippo pathway, or an agent that acts downstream of disrupted Hippo signaling at the concentrations previously indicated, for a transient period of time of at least about 1 hour to about 24 hours, and may be from about 6 to about 12 hours. The concentrations may be adjusted to reflect the potency of the agent(s).

Following follicle maturation, the oocytes present in the follicles may be utilized for *in vitro* purposes. In some disclosure herein the oocytes are utilized directly, and in others the follicles are contacted with one or more factors to modulate the oocyte maturation, *e.g.* the cultures may be contacted with a concentration of FSH or FSH analog sufficient to induce oocyte maturation *in vitro,* where the FSH or FSH analog may be recombinant, modified, native, *etc.* Following *in vitro* maturation the oocytes may be fertilized *in vitro* for implantation; may be fertilized *in vitro* for generation of stem cell lines; may be utilized without fertilization for various research purposes, and the like.

The follicles may be additionally cultured in the presence of one or more of c-kit ligand (Hutt et al., 2006; Parrott and Skinner, 1999), neurotrophins (Ojeda et al., 2000), vascular endothelial growth factor (Roberts et al., 2007), bone morphogenetic protein (BMP)-4 (Tanwar et al., 2008), BMP7 (Lee et al., 2001), leukemia inhibitory factor (Nilsson et al., 2002), basic FGF (Nilsson et al., 2001), keratinocyte growth factor (Kezele et al., 2005), and the like, where the factor(s) may be added in conjunction with an agent that disrupts signaling in the Hippo pathway, or an agent that acts downstream of disrupted Hippo signaling, or following exposure to one or both of an agent that disrupts signaling in the Hippo pathway, or an agent that acts downstream of disrupted Hippo signaling. For example, an LH agonist, including eCG and/or HCG may be administered following oocyte maturation by FSH.

In other disclosure herein the follicles may be transplanted to an animal recipient for maturation. As described above, methods are known in the art for transplantation of ovaries or fragments thereof at an ovarian site, at a kidney site, at a sub-cutaneous site, etc. are known in the art and may find use. Where the ovarian tissue is transplanted to an ovary, fertilization may proceed without additional *in vitro* manipulation. Where the ovarian tissue is transplanted to a non-ovarian site, *e.g.* a sub-cutaneous site, the oocytes may be subsequently removed for in vitro fertilization. The recipient may provide endogenous FSH for maturation of the oocytes, or may be provided with exogenous FSH or FSH analog for that purpose, including recombinant, long-acting FSH-CTP, and the like.

In other disclosure herein, the exposure is performed *in vivo,* locally to the ovary or systemically administered to an individual. The data obtained from cell culture and/or animal studies can be used in formulating a range of dosages for humans. The dosage of the active ingredient typically lines within a range of circulating concentrations that include the ED₅₀ with little or no toxicity. The dosage can vary within this range depending upon the dosage form employed and the route of administration utilized. The individual is typically contacted with an effective concentration for at least about 6 hours, usually at least about 12 hours, and may be for at least about 1 day and not more than about one week, usually not more than about 3 days.

The compositions can also include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the *in vivo* stability of the polypeptide, or otherwise enhance its pharmacological properties (*e.g.,* increase the half-life of the polypeptide, reduce its toxicity, enhance solubility or uptake). Examples of such modifications or complexing agents include sulfate, gluconate, citrate and phosphate. The polypeptides of a composition can also be complexed with molecules that enhance their *in vivo* attributes. Such molecules include, for example, carbohydrates, polyamines, amino acids, other peptides, ions (e.g., sodium, potassium, calcium, magnesium, manganese), and lipids.

Further guidance regarding formulations that are suitable for various types of administration can be found in Remington's Pharmaceutical Sciences, Mace Publishing Company, Philadelphia, PA, 17th ed. (1985). For a brief review of methods for drug delivery, see, Langer, Science 249:1527-1533 (1990).

The effective dose of at least one of an agent that disrupts signaling in the Hippo pathway, or an agent that acts downstream of disrupted Hippo signaling can be administered in a variety of different ways. Examples include administering a composition via oral, topical, intraperitoneal, intravenous, intramuscular, subcutaneous, subdermal, transdermal, intra-ovarian methods. In pharmaceutical dosage forms, the compounds may be administered in the form of their pharmaceutically acceptable salts, or they may also be used alone or in appropriate association, as well as in combination with other pharmaceutically active compounds.

The term "unit dosage form," as used herein, refers to physically discrete units suitable as unitary dosages for human and animal subjects, each unit containing a predetermined quantity of compounds of the present disclosure calculated in an amount sufficient to produce the desired effect in association with a pharmaceutically acceptable diluent, carrier or vehicle. The specifications for the novel unit dosage forms of the present disclosure depend on the particular compound employed and the effect to be achieved, and the pharmacodynamics associated with each compound in the host.

Typical dosages for systemic administration range from 0.1 µg to 100 milligrams per kg weight of subject per administration. A typical dosage may be one tablet taken from two to six times daily, or one time-release capsule or tablet taken once a day and containing a proportionally higher content of active ingredient. The time-release effect may be obtained by capsule materials that dissolve at different pH values, by capsules that release slowly by osmotic pressure, or by any other known means of controlled release.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

Following such exposure, the individual may be treated with recombinant FSH or FSH analogs, including, without limitation, naturally occurring FSH in an *in vivo* host animal, FSH analogs such as FSH-CTP, single chain analogs, pegylated FSH, and the like, at a concentration that is effective to release a mature oocyte. The individual may also be treated with an LH agonist as described above. Alternatively, the oocytes may be removed from the ovary and utilized for in vitro manipulation as described above.

### EXPERIMENTAL

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (*e.g.* amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

### Example 1

### Ovarian fragmentation disrupts Hippo signaling to promote follicle growth

Primary ovarian insufficiency (POI) and polycystic ovarian syndrome (PCOS) are ovarian diseases causing infertility. Although there is no effective treatment for POI, therapies for PCOS include ovarian wedge resection or laser drilling to induce follicle growth. Underlying mechanisms for these disruptive procedures are unclear. Here, we explored the role of the conserved Hippo signaling pathway that serves to maintain optimal size across organs and species. We found that fragmentation of murine ovaries promoted actin polymerization and disrupted ovarian Hippo signaling, leading to increased expression of downstream growth factors, promotion of follicle growth, and the generation of mature oocytes. In addition to elucidating mechanisms underlying follicle growth elicited by ovarian damage, we further demonstrated additive follicle growth when ovarian fragmentation was combined with Akt-stimulator treatments. The ovarian fragmentation-in vitro activation approach is not only valuable for treating infertility of POI patients but is useful for middle-aged infertile women, cancer patients undergoing sterilizing treatments, and other conditions of diminished ovarian reserve.

Between 5-10% of reproductive age women are infertile due to polycystic ovarian syndrome (PCOS) whereas 1% of them suffer from infertility due to primary ovarian insufficiency (POI). They are infertile due to aberrant follicle growth. As early as the 1930s, ovarian wedge resection was used for PCOS treatment to induce follicle growth, followed by recent success based on ovarian 'drilling' by diathermy or laser. In addition, ovarian cortices are routinely fragmented to allow better freezing and grafting for fertility preservation in cancer patients who underwent sterilizing treatment. Subsequent auto-transplantation of ovarian fragments is associated with spontaneous follicle growth. Underlying mechanisms for these disruptive procedures to promote follicle growth are, however, unclear.

The Hippo signaling pathway is essential to maintain optimal organ size and is conserved in all metazoan animals. Hippo signaling consists of several negative growth regulators acting in a kinase cascade that ultimately phosphorylates and inactivates key Hippo signaling effectors, YAP (Yes-associated protein)/TAZ (transcriptional co-activator with PDZ-binding motif). When Hippo signaling is disrupted, decreases in YAP phosphorylation increase nuclear levels of YAP. YAP acts in concert with TEAD transcriptional factors to increase downstream CCN growth factors and BIRC apoptosis inhibitors. CCN proteins, in turn, stimulate cell growth, survival, and proliferation.

Using a murine model, we now demonstrated the promotion of follicle growth following ovarian fragmentation and allotransplantation. Ovarian fragmentation increased actin polymerization, decreased phospho-YAP levels, increased nuclear localization of YAP as well as enhanced expression of CCN growth factors and BIRC apoptosis inhibitors. Fragmentation-induced follicle growth was partially blocked by CCN2 antibodies and verteporfin, a small molecule that inhibits interactions of YAP with TEAD transcriptional factors. Studies using PTEN deletion mice indicated the stimulatory roles of Akt signaling in the development of primordial and secondary follicles.

### Results

Ovarian fragmentation promoted follicle growth: We fragmented ovaries from juvenile (day 10) mice containing secondary and smaller follicles, followed by allo-transplantation under kidney capsules of adult hosts. As shown in Fig. 1A, major increases in graft sizes were evident after cutting ovaries into three pieces and grafting for 5 days as compared with paired intact ovaries. Graft weights increased after cutting ovaries into 2 to 4 pieces or incubating fragments for up to 24h before grafting (Fig. 1B). Histological analyses (Fig. 4A) and follicle counting of grafts (Fig. 1C and 4B) indicated a loss of total follicles following fragmentation/grafting. However, major increases in the percentage of late secondary, and antral/preovulatory follicles were evident, accompanied by decreases in primordial follicles (Fig. 1C). As compared to day 10 ovaries, the grafting procedure led to decreases in absolute number of primordial, primary, and early secondary follicles (Fig. 4B). Furthermore, cutting/grafting of ovaries from older mice, including those containing early antral follicles from day 23 animals, also increased graft weights (Fig. 1D).

After grafting for 5 days, hosts received an ovulating dose of hCG. As shown in Fig. 4C, numbers of oocytes retrieved from fragmented grafts per ovary were 3.1-fold of those from intact grafts, accompanied by increased percentages of mature oocytes. Mature oocytes retrieved from fragmented grafts were fertilized and their development to early embryos was comparable to controls. After embryo transfer, healthy pups were delivered (Fig. 4D). Similar to mouse studies, fragmentation/autotransplantation of ovaries from rats also increased graft weights (Figs. 4E, F).

Ovarian fragmentation increased actin polymerization and disrupted Hippo signaling: Real-time RT-PCR and immunoblotting analyses (Figs. 5A, B) indicated the expression of transcripts and proteins for key Hippo signaling genes in ovaries of juvenile mice. Also, immunohistochemical staining of ovaries from adult mice (Fig. 5C) indicated the expression of MST1/2, SAV1, LAT 4/2, and TAZ mainly in the cytoplasm of granulosa cells, theca cells, and oocytes of follicles at all sizes but at lower levels in the corpus luteum.

Polymerization of globular actin (G-actin) to the filamentous form (F-actin) is important for cell shape maintenance and locomotion. Recent genome-wide RNAi screening demonstrated that induction of extra F-actin formation disrupted Hippo signaling and induced overgrowth in *Drosophila* imaginal discs and human HeLa cells. As shown in Fig. 2A, a transient increase in ratios of F-actin to G-actin was detected at 1h after ovarian fragmentation. The Hippo signaling kinase cascade phosphorylates YAP to promote its cytoplasmic localization and degradation, thus decreasing its transcriptional actions. When Hippo signaling is disrupted, decreases in phospho-YAP (pYAP) increase nuclear YAP levels. After ovarian fragmentation and incubation for 1h, decreases in pYAP levels and pYAP to total YAP ratios were evident (Fig. 2B), suggesting Hippo signaling disruption. In intact ovaries from day 10 mice, immunohistochemical staining indicated that YAP was localized in the cytoplasm of granulosa cells in most follicles at primary and secondary stages (Fig. 5D). At 4h after fragmentation, nuclear staining of YAP was found in granulosa cells of primary and secondary follicles.

Disruption of Hippo signaling leads to increased expression of downstream CCN growth factors and BIRC apoptosis inhibitors. As shown in Fig. 2C, ovarian fragmentation and subsequent grafting increased transcript levels for several CCN growth factors (CCN2, 3, 5, and 6) and apoptosis inhibitors (BIRC1 and 7) in fragmented ovaries. Similar changes were found following continuous culture without grafting (Fig. 6A). Immunoblotting of highly expressed CCN2 demonstrated increased CCN2 proteins in fragmented ovaries (Fig. 2D). Real-time RTPCR analyses showed fragmentation-induced increases in CCN2 transcripts in somatic cells, but not oocytes (Fig. 6B).

The ability of CCN proteins to promote ovarian growth was further demonstrated by dose-dependent increases in ovarian explant weights after culturing with CCN2, 3, 5, and 6 (Fig. 2E). Analyses of follicle dynamics indicated the ability of CCN factors to promote the development of primary follicles to the late secondary stage in ovarian explants (Fig. 6C), underscoring the role of CCN proteins as ovarian growth factors.

Roles of Hippo signaling and CCN2 in fragmentationinduced follicle growth: YAP has no transcriptional activity and its actions are dependent on downstream transcriptional factors. Recent drug library screening identified a small molecule verteporfin, capable of inhibiting YAP association with TEAD transcriptional factors and suppressing YAP-induced liver overgrowth. Because fragmentation-induced CCN and BIRC changes were transient, we injected day 10 mice for 3h with verteporfin before obtaining ovaries for fragmentation. As shown in Fig. 7A, pretreatment with verteporfin blocked fragmentation-induced increases in CCN2 transcripts without affecting those for anti-Müllerian hormone (AMH), a secondary follicle marker.

In contrast to graft weight increases found between intact and fragmented ovarian pairs from vehicle pretreated animals, no significant changes in graft weights were found between intact and fragmented pairs after pretreatment with verteporfin (Fig. 7B). Follicle counting of grafts indicated no loss of total follicles with verteporfin pretreatment (Fig. 7C). In contrast, verteporfin pretreatment prevented fragmentation induced increases in late secondary follicles with smaller suppression of antral/preovulatory follicles. We further incubated ovarian fragments with CCN2 antibodies for 18h before grafting. Neutralization of endogenous CCN2 suppressed fragmentation induced graft weight gain by 75% (Fig. 7D). These findings underscore the role of Hippo signaling in fragmentation-induced follicle growth.

Additive effects of Hippo signaling disruption and Akt stimulation on secondary follicle growth: In addition to the stimulatory role of Akt signaling in primordial follicle development, conditional deletion of the PTEN gene in granulosa cells of secondary follicles also promoted follicle growth. We isolated secondary follicles from juvenile mice and demonstrated the ability of Akt stimulating drugs (PTEN inhibitor and PI3K activator) to promote secondary follicle growth (Fig. 3A). We further tested combined effects of Akt stimulating drugs and Hippo signaling disruption on ovarian graft growth. Using ovaries obtained from day 10 mice containing secondary and smaller follicles, we found additive increases in ovarian graft weights when fragmented ovaries were incubated with Akt stimulating drugs followed by grafting (Fig. 3B). Counting of follicles indicated increases in late secondary and antral/preovulatory follicles induced by fragmentation and Akt stimulation (Fig. 3C and 8).

We obtained human ovarian cortical cubes containing secondary and smaller follicles. RT-PCR analyses demonstrated the expression of key Hippo signaling genes (Fig. 9A) whereas immunohistochemical analyses showed the expression of SAV1, LAT 4/2, YAP and TAZ in granulosa cells, theca cells, and oocytes of primordial to secondary follicles (Fig. 9B). We then thawed cryo-preserved human ovarian cortical strips (1-2 mm thickness and 1x1 cm) and cut them into small cubes (1-2 mm²) before incubation. Real-time RT-PCR analyses indicated time dependent increases in transcript levels for CCN2, 3, 5 and 6 (Fig. 9C). Higher CCN growth factor expression was found in ovarian cubes after further fragmentation from strips, suggesting fragmentation-induced disruption of Hippo signaling. We then cut human cortical strips containing secondary and smaller follicles (Fig. 3D) and incubated them with Akt stimulators before xeno-grafting into immune-deficient mice. Within 4 weeks, antral follicles were detected, demonstrating rapid follicle growth (Figs. 3E, F).

Findings across multiple organ systems and model organisms have implicated Hippo signaling in the maintenance of organ sizes. However, our results uniquely document a role for Hippo signaling in mammalian ovaries. Our data indicate that ovarian fragmentation increased actin polymerization and disrupted Hippo signaling by decreasing pYAP levels together with increased nuclear localization of YAP, leading to increased expression of CCN growth factors and BIRC apoptosis inhibitors. Secreted CCN2 and related factors promoted follicle growth after transplantation (Fig. 10).

It is clear that most ovarian follicles are constrained to growth under physiological conditions due to local Hippo signaling. Consistent with the role of Hippo signaling genes in restraining ovarian follicle growth, specific deletion of SAV1 or MST1/2 genes in hepatocytes resulted in enlarged livers. Likewise, conditional deletion of SAV1 led to enlarged hearts. Hippo signaling is also critical for tissue regeneration and expansion of tissue-specific progenitor cells. For the ovary, LAT 4 null female mice exhibited a POI phenotype whereas LAT 4 regulates the transcriptional activity of FOXL2, a gene mutated in some POI patients (Fig. 10, boxed). Genome-wide association studies also implicated YAP as a susceptibility gene for PCOS whereas deletion of CCN2/CTGF in ovarian granulosa cells in mice led to subfertility and aberrant follicle development Also, genome-wide analyses identified changes in gene copy numbers for BIRC1 in POI patients.

F-actin formation in the stress fiber is required for the disruption of Hippo signaling and nuclear YAP accumulation. F-actin probably functions as a scaffold for Hippo signaling components because Hippo signaling genes MST1/2, merlin, and Amot all bind to actin. The upstream diaphanous (DIAPH) genes accelerate actin nucleation and suppress actin depolymerization. Of interest, disruption of the DIAPH2 coding region was found in a POI family whereas genome-wide association studies identified DIAPH2 and DIAPH3 as candidate genes in regulating follicle reserve and menopause (Fig. 10). Intestinal damage using dextran sodium sulfate decreases pYAP to total YAP ratios in regenerating crypts. Also, CCN1/CYR61 was induced in proximal straight tubules following ischemic reperfusion injury of the kidney. In the obstructed bladder, expression of CCN2/CTGF and CCN1/CYR61 were also induced.

Disruption of Hippo signaling following actin polymerization likely represents a general mechanism in regulating tissue damage and remodeling, linking mechanical alterations of structural components to intracellular signaling. Changes in actin polymerization and downstream events induced by ovarian fragmentation were transient in nature and increases in CCN2/3/5/6 transcript levels occurred even when frozen human ovarian strips were fragmented after thawing.

CCN growth factors and apoptosis inhibitors likely induce additional downstream changes, including the PI3K-TOR signaling pathway, to promote follicle growth. Although vascularization changes during grafting cannot be ruled out, treatment with CCN2 antibodies or verteporfin partially suppressed fragmentation-induced increases in graft weights, underscoring the role of Hippo signaling. Mechanical tension associated with the rigid sclerotic capsules in some PCOS ovaries could lead to arrested follicle development. Ovarian wedge resection or 'drilling' by diathermy/laser in PCOS patients results in follicle growth and comparable live birth rate as compared to the popular gonadotropin treatment. Our studies show that damage incurred by cutting or drilling PCOS ovaries can enhance actin polymerization and disrupt Hippo signaling to promote follicle growth. Local administration of actin polymerization drugs or CCN growth factors could provide new treatments for PCOS patients and minimize follicle loss associated with ovarian damage.

Conditional deletion of the PTEN gene in granulosa cells of secondary follicles in mice promoted follicle growth. We demonstrated additive increases in ovarian graft weights and follicle growth following Hippo signaling disruption (fragmentation) and Akt stimulation (treatment with PTEN inhibitors and PI3K activators). Using the present IVA protocol, rapid growth of human secondary follicles to the antral stage was found in immune deficient mice. Although the exact stage of residual follicles in individual POI ovaries is unclear, we generated preovulatory follicles from several patients in a few weeks. The present approach represents a new infertility therapy for POI patients.

In addition to POI, the present approach can be used for fertility preservation in cancer patients undergoing sterilizing treatments, and other conditions of diminished ovarian reserve. Although menopause occurs at an average of 51 years of age, many middle-aged women between 40 and 45 years of age suffer from aging-associated infertility. Because their ovaries still contain secondary and smaller follicles, our approach should be effective. Without overcoming age- or environment-related increases in genetic defects in oocytes, the present approach provides more mature oocytes for embryonic development.

### Methods

Animals: CD-1 and B6D2F1 mice were purchased from Charles River Laboratories (Wilmington, MA) and housed in the animal facility of Stanford University under 12h dark/light with free access to food and water. Mice were treated in accordance with guidelines of the local Animal Research Committee.

Patient samples: Human ovarian cortical cubes were obtained from three patients (32, 33, and 36 years of age) with benign ovarian tumor but exhibiting normal menstrual cycles. In addition, cortical cubes (5x5 mm) were obtained from Caesarean-section patients (23-37 years of age) not planning for future pregnancy. Informed consent from patients and approval from the Human Subject Committee of Akita University were obtained. All samples were immediately stored at -70C. Cortical cubes containing primordial, primary, and secondary follicles from patients with tumors were used for RT-PCR or immunohistochemical staining to determine the expression of Hippo signaling genes. Cortical cubes from C-section patients were used to evaluate the expression of CCN growth factors after fragmentation.

Ovarian fragmentation and grafting: Paired ovaries from CD-1 mice at different ages were excised in L-15 medium. One ovary from each animal was cut into 2-4 pieces, whereas the contralateral one remained intact. Ovaries were transferred to culture plate inserts (Millipore, Bedford, MA) and cultured in MEMα medium with 3 mg/ml BSA, 0.23 mM sodium pyruvate, 50 ug/ml vitamin C, 30 mIU/ml FSH, 50 mg/L streptomycin sulfate, and 75 mg/L penicillin G. After 30 min. of incubation, media were replaced and incubated for another 30 min. At the end of incubation, paired ovaries (intact and fragmented) from the same donor were inserted under the kidney capsule of the same adult ovariectomized hosts (9-10 weeks of age). Hosts were injected with 1 IU FSH daily starting from the day after transplantation for 5-7 days. At the end of transplantation, grafts were collected for fixation before weight determination and histological analyses. For testing the effect of actin polymerization, paired ovaries from day 10 mice were either incubated with or without JASP for 30 min., followed by measurement of F-actin and G-actin levels and CCN2 expression. Some paired ovaries were grafted into adult hosts for 5 days before measurement of graft weights. For rat studies, ovaries from day 10 rats were fragmented and cultured for 1h before auto-transplantation into kidneys of the same animals for 5 days.

Follicle counting: Ovarian grafts were collected and fixed in 10% buffered formalin overnight, embedded in paraffin, serially sectioned at 8 um and stained with hematoxylin and eosin. Only follicles with clearly stained oocyte nucleus were counted as described.

IVF and embryo transfer: At 5 days after transplantation, B6D2F1 host mice were treated with 10 IU eCG for 48h, followed by a single injection of hCG (10 IU) to induce oocyte maturation. Twelve h later, grafted ovarian fragments were collected and oocytes associated with expanded cumulus cells were retrieved by puncturing the grafts in M2 medium (Millipore). As controls, B6D2F1 female mice at day 23 of age were primed with eCG for 48h, followed by a single injection of hCG to induce ovulation. For IVF, sperm from B6D2F1 male mice were collected into human tubal fluid media (Millipore) and pre-incubated for 1h at 37C as described. Oocytes were fertilized with sperm (2-3 X 10⁵/ml) for 6h and inseminated oocytes were transferred to KSOM-AA medium (Millipore) to allow for development into blastocysts. In addition, some two-cell embryos obtained from ovarian grafts after ovarian fragmentation were transferred into oviducts of pseudopregnant, 8-week-old CD1 mice pre-mated with vasectomized males of the same strain. Pregnancy was monitored and pups delivered.

Immunoblotting analysis: Proteins from NIH3T3 cells or ovaries of mice at day 10 of age were extracted using M-PER Mammalian Protein Extraction Reagent (Thermo, Rockford, IL) containing a protease inhibitor cocktail (Thermo). Proteins were loaded on 4-15% SDS-polyacrylamide gels (Bio-Rad Laboratories, Hercules, CA) and electroblotted into Hybond-P membranes (GE Healthcare, Piscataway, NJ). After blocking for 1h at room temperature, membranes were incubated with first antibodies obtained from Cell Signaling Technology (Beverly, MA), except for the CCN2 antibody from Santa Cruz Biotechnology (Santa Cruz, CA). This was followed by incubation with secondary antibodies. Signals were developed using enhanced chemiluminescence (ECL Kit, GE Healthcare) and quantitated using a densitometer.

Real-time RT-PCR analyses: To detect the expression of Hippo signaling genes, ovaries from mice at day 10 of age were obtained to determine transcript levels for Mer, Ex, MST1, MST2, SAV1, Mob1a, Mob1b, LATS1, LATS2, YAP, and TAZ. For studies on the expression of CCN growth factors, paired ovaries from day 10 mice were obtained at different times after incubation and grafting or after culture alone. Total RNAs from ovaries or grafts were extracted using an RNeasy Micro Kit (QIAGEN Sciences, Valencia, CA) and cDNAs were synthesized using a Sensicript RT Kit (QIAGEN) according to the manufacture's protocol. Real-time PCR was performed using iTaq SYBR Green SuperMix (Bio-Rad) on a Smart Cycler TD system (Cepheid) as follows: 15 min. at 95C, 45 cycles of 15 sec. at 95C, and 60 sec. at 60C. Relative abundance of specific transcripts was normalized to relative abundance of β-actin levels.

Human ovarian cortical fragments from patients with benign ovarian tumor were used for RT-PCR of Hippo signaling genes. To determine changes in CCN growth factor expression after fragmentation, ovarian samples from C-section patients were used. After thawing, ovarian cubes were further cut into 4 fragments (2x2 mm) and washed 3 times in L-15 medium. Ovarian fragments were transferred to culture plate inserts and cultured for 0, 1, and 3h in MEMα medium with 10% human serum albumin, 1% antibiotic/anti-mycotic solution, 0.3 IU/ml FSH. Culture medium was replaced with fresh ones at 30 min. intervals. After incubation, samples were washed 3 times in PBS, and subjected to real-time quantitative RT-PCR for transcript levels of CCN growth factors. To normalize basal levels among patients, data were expressed as fold changes relative to the 0h data expressed as 1.0.

Ovarian explant cultures: Ovaries from day 10 mice were placed on culture plate insert (Millipore) and cultured in DMEM/F12 containing 0.1% BSA, 0.1% Albumax II, insulin-transferrin-selenium, 0.05 mg/ml L-ascorbic acid and penicillin-streptomycin for 4 days with medium changes after 2 days of culture as described. At the end of culture, ovarian weight was measured and some of the ovaries were embedded for histological analyses of follicle dynamics.

Measurement of F-actin levels: Ratios of F-actin to G-actin in intact and fragmented ovaries were determined by F-actin/G-actin in vivo assay kit (Cytoskeleton, Denver, CO). After incubation for 1h, intact or fragmented ovaries were homogenized in the F-actin stabilization buffer containing 50 mM PIPES PH 6.9, 50 mM KCI, 5 mM MgCl₂, 5 mM EGTA, 5% glycerol, 0.1% Nonidet P40, 0.1% Triton X-100, 0.1% Tween 20, 0.1% 2-mecraptoethanol, 0.0001% AntifoamC, 1mM ATP, 0.0004 mM tosyl arginine methyl ester, 0.015 mM leupeptin, 0,001 mM pepstatin A, and 0.001 M bezamidine. After incubation at 37C for 10 min., the lysate was centrifuged at 350xg for 5 min. to remove unbroken tissue debris. After further centrifugation at 100,000x g for 1h at 37C, the supernatant was collected. Pellets were resuspended in ice-cold molecular grade water containing 8M urea and incubated on ice for 1h with gentle mixing every 15 min. To measure F/G-actin ratios, equal amounts of supernatant (G-actin) and resuspended pellet (F-actin) were subjected to immunoblotting analysis using the pan-actin antibody (Cytoskeleton).

Immunostaining analyses: Immuno-histochemical staining of SAV1, LATS1/2, YAP, and TAZ were performed using anti-SAV1 (Proteintech, Chicago IL), anti-LATS1/2 (Abnova, Walnut, CA), anti-YAP (Cell Signaling Technology), or anti-TAZ (Cell Signaling Technology) antibodies at 1:100, 1:50, 1:200, or 1:200 dilution, respectively. For mouse MST1/2, primary antibodies were from AbFRONTIER (Seoul, Korea) and used at 1:100 dilution. After deparaffinization and dehydration, antigen retrieval was performed by autoclave heating at 121C for 10 min. in 10 mM citrate buffer (pH 6) (3 min. for three times). Endogenous peroxidase activities were quenched with 0.3% hydrogen peroxidase in methanol for 30 min. After blocking with 10% BSA-Tris-buffered saline (Sigma) for 30 min., slides were incubated with primary antibodies overnight at 4C. After three washes in Tris-buffered saline, slides were incubated with biotinylated secondary antibodies (Invitrogen) for 30 min. at room temperature. After three washes, bound antibodies were visualized using a Histostain SP kit (Invitrogen). For negative controls, the primary antibody was replaced by nonimmune IgG (Dako).

Statistical analyses: Results are presented as the mean+/- SEM of three or more independent assays. Statistical significance was determined by using one-way ANOVA, followed by Fisher's protected significant difference test with P<0.05 being statistically significant.

### Example 2

### S1P Hippo signaling

We tested the ability of S1P to promote follicle growth. As shown in Fig. 12 (left panel), addition of S1P (6 µM) to explant cultures of ovaries obtained from day 10 mice for 4 days led to major increases in ovarian weights. In addition, culturing day 10 ovaries with S1P (6 µM) for 18h, followed by grafting into adult ovariectomized hosts for 5 days also led to increases in graft weights (Fig. 12, right panel). When some ovaries were treated with S1P plus the IVA drugs (PTEN inhibitors and PI3K stimulator), additive increases in graft weights were found. We hypothesized that S1P acts through its receptors in granulosa cells to trigger actin polymerization, disrupt Hippo signaling and promote follicle growth.

Oocyte apoptosis induced by X-irradiation is suppressed by intrabursal treatment with S1P (Morita et al. (2000) Nature Med. 6, 1109-1114). In vivo delivery of FTY720 (a S1P mimetic) using intraovarian cannulation prevents radiation-induced ovarian failure and infertility in adult female nonhuman primates (Zelinski et al. (2011) Fertility and sterility 95, 1440-1445. e1447). FTY720 (trade name Gilenya, Novartis) is an immuno-modulating drug, approved for treating multiple sclerosis.

Demonstration of the ability of S1P and its agonists to disrupt ovarian Hippo signaling and promote follicle growth provides the basis for use of an approved, low toxicity, drug for the treatment of the prevalent PCO syndrome. S1P or its mimetic is delivered locally into polycystic ovaries to promote follicle growth, thus replacing the present use of damaging procedures (wedge resection and laser drilling).

## Claims

1. A method of promoting the development of mature oocytes, the method comprising:
contacting ovarian tissue *in vitro* with an effective dose of both a first agent that disrupts signaling in the Hippo pathway, the agent being selected from jasplakinolide (JASP) and sphingosine 1-phosphate (S1P); and a second agent that acts downstream of disrupted Hippo signaling, the agent being a CCN protein selected from CCN 2, 3, 5 and 6, in a dose and for a period of time sufficient to promote development of a mature oocyte.

2. The method of Claim 1, wherein the mature oocyte is contained within a pre-ovulatory follicle.

3. The method of Claim 2, wherein the pre-ovulatory follicle is a secondary follicle.

4. The method of Claim 2 wherein the pre-ovulatory follicle is an antral follicle.

5. The method of any one of Claims 1-4, wherein the ovarian tissue is human.

6. The method of Claim 5, wherein the ovarian tissue is contacted for a period of one hour to four days.

7. The method of Claim 6, further comprising following the contacting step, performing a step of contacting the ovarian tissue with follicle-stimulating hormone (FSH) or an analog thereof in a dose and for a time effective to promote development of a mature oocyte.

8. The method of Claim 1, further comprising following the contacting step, performing a step of harvesting the ovarian tissue.

9. The method of Claim 5, wherein said contacting step further comprises contacting the ovarian tissue with an effective dose of at least one of a PTEN inhibitor and a PI3 kinase activator.

10. A composition comprising an agent that disrupts signaling in the Hippo pathway and an agent that acts downstream of disrupted Hippo signaling for use in the treatment of mammalian female infertility,
wherein the agent that disrupts signaling in the Hippo pathway is selected from jasplakinolide (JASP) and sphingosine 1-phosphate (S1P); and
the agent that acts downstream of disrupted Hippo signaling is a CCN protein selected from CCN 2, 3, 5 and 6.

11. A composition for use according to Claim 10 wherein the female infertility is due to a condition selected from the group consisting of premature ovarian failure, perimenopause, FSH low responsiveness, polycystic ovarian syndrome, diminished ovarian reserve and age-related infertility.

12. A composition for use according to Claim 10, wherein the mammalian female is a human.

13. A composition for use according to Claim 10, wherein the treatment further comprises transplantation of the activated follicles obtained by the method according to claims 2 or 4 to an *in vivo* recipient.

14. A composition for use according to Claim 13, wherein the treatment further comprises administering an LH agonist to said recipient following implantation.

## Patentansprüche

1. Verfahren zur Förderung der Entwicklung von reifen Oocyten, wobei das Verfahren Folgendes umfasst:
Kontaktieren von Ovarialgewebe in vitro mit einer wirksamen Menge von sowohl einem ersten Mittel, das die Signalgebung in dem Hippo-Signalweg unterbricht, wobei das Mittel aus Jasplakinolid (JASP) und Sphingosin-1-phosphat (S1P) ausgewählt ist; als auch einem zweiten Mittel, das stromab von unterbrochener Hippo-Signalgebung agiert, wobei das Mittel ein CCN-Protein ist, ausgewählt aus CCN 2, 3, 5 und 6, in einer Dosierung und über eine Zeitspanne hinweg, die ausreichend sind, um die Entwicklung einer reifen Oocyte zu fördern.

2. Verfahren nach Anspruch 1, wobei die reife Oocyte in einem präovulatorischen Follikel enthalten ist.

3. Verfahren nach Anspruch 2, wobei der präovulatorische Follikel ein sekundärer Follikel ist.

4. Verfahren nach Anspruch 2, wobei der präovulatorische Follikel ein antraler Follikel ist.

5. Verfahren nach einem der Ansprüche 1 bis 4, wobei das Ovarialgewebe menschlich ist.

6. Verfahren nach Anspruch 5, wobei das Ovarialgewebe über eine Zeitspanne von 1 Stunde bis 4 Tagen hinweg kontaktiert wird.

7. Verfahren nach Anspruch 6, das weiters nach dem Kontaktierungsschritt das Durchführen eines Schritts des Kontaktierens des Ovarialgewebes mit follikelstimulierendem Hormon (FSH) oder einem Analogon davon in einer Dosierung oder über Zeitspanne hinweg umfasst, die wirksam sind, um die Entwicklung einer reifen Oocyte zu fördern.

8. Verfahren nach Anspruch 1, das weiters nach dem Kontaktierungsschritt das Durchführen eines Schritts der Abnahme von Ovarialgewebe umfasst.

9. Verfahren nach Anspruch 5, wobei der Kontaktierungsschritt weiters das Kontaktieren des Ovarialgewebes mit einer wirksamen Menge von zumindest einem von einem PTEN-Hemmer und einem PI3-Kinaseaktivator umfasst.

10. Zusammensetzung, die ein Mittel, das die Signalgebung in dem Hippo-Signalweg unterbricht, und ein Mittel, das stromab von unterbrochener Hippo-Signalgebung agiert, umfasst, zur Verwendung bei der Behandlung von Unfruchtbarkeit bei weiblichen Säugetieren,
wobei das Mittel, das die Signalgebung in dem Hippo-Signalweg unterbricht, aus Jasplakinolid (JASP) und Sphingosin-1-phosphat (S1P) ausgewählt ist; und
das Mittel, das stromab von unterbrochener Hippo-Signalgebung agiert, ein CCN-Protein, ausgewählt aus CCN 2, 3, 5 und 6, ist.

11. Zusammensetzung zur Verwendung nach Anspruch 10, wobei die weibliche Unfruchtbarkeit auf eine Bedingung, ausgewählt aus der aus vorzeitigem Versagen der Ovarien, Perimenopause, niedriger FSH-Reaktionsfähigkeit, polyzystischem Ovarialsyndrom, verminderter Ovarialreserve und altersbedingter Unfruchtbarkeit bestehenden Gruppe, zurückzuführen ist.

12. Zusammensetzung zur Verwendung nach Anspruch 10, wobei das weibliche Säugetier ein Mensch ist.

13. Zusammensetzung zur Verwendung nach Anspruch 10, wobei die Behandlung weiters die Transplantation der durch das Verfahren nach den Ansprüchen 2 oder 4 erhaltenen aktivierten Follikel in einen In-vivo-Empfänger umfasst.

14. Zusammensetzung zur Verwendung nach Anspruch 13, wobei die Behandlung weiters das Verabreichen eines LH-Agonisten an den Empfänger nach der Implantation umfasst.

## Revendications

1. Procédé de promotion du développement d'ovocytes matures, le procédé comprend l'étape consistant à :
mettre en contact *in vitro* du tissu ovarien avec une dose efficace à la fois d'un premier agent qui perturbe une signalisation dans la voie Hippo, l'agent étant choisi parmi du jasplakinolide (JASP) et du 1-phosphate de sphingosine (S1P) ; et un second agent qui agit en aval de la signalisation Hippo perturbée, l'agent étant une protéine CCN choisie parmi CCN 2, 3, 5 et 6, à une dose et pendant une durée suffisantes pour favoriser le développement d'un ovocyte mature.

2. Procédé selon la revendication 1, dans lequel l'ovocyte mature est contenu dans un follicule pré-ovulatoire.

3. Procédé selon la revendication 2, dans lequel le follicule pré-ovulatoire est un follicule secondaire.

4. Procédé selon la revendication 2, dans lequel le follicule pré-ovulatoire est un follicule antral.

5. Procédé selon l'une quelconque des revendications 1 à 4, dans lequel le tissu ovarien est humain.

6. Procédé selon la revendication 5, dans lequel le tissu ovarien est mis en contact pendant une période d'une heure à quatre jours.

7. Procédé selon la revendication 6, comprenant en outre à la suite de l'étape de mise en contact, l'exécution d'une étape consistant à mettre en contact le tissu ovarien avec une hormone folliculo-stimulante (FSH) ou un analogue de celle-ci en une dose et pendant un temps efficace pour favoriser le développement d'un ovocyte mature.

8. Procédé selon la revendication 1, comprenant en outre à la suite de l'étape de mise en contact, l'exécution d'une étape consistant à récolter le tissu ovarien.

9. Procédé selon la revendication 5, dans lequel ladite étape de mise en contact comprend en outre la mise en contact du tissu ovarien avec une dose efficace d'au moins un parmi un inhibiteur de PTEN et un activateur de PI3 kinase.

10. Composition comprenant un agent qui perturbe la signalisation dans la voie Hippo et un agent qui agit en aval de la signalisation Hippo perturbée pour une utilisation dans le traitement de l'infertilité féminine chez les mammifères,
dans laquelle l'agent qui perturbe la signalisation dans la voie Hippo est choisi parmi du jasplakinolide (JASP) et du 1-phosphate de sphingosine (S1P); et
l'agent qui agit en aval de la signalisation Hippo perturbée est une protéine CCN choisie parmi CCN 2, 3, 5 et 6.

11. Composition à utiliser selon la revendication 10, dans laquelle l'infertilité féminine est due à une affection choisie dans le groupe comprenant une insuffisance ovarienne prématurée, une périménopause, une faible réactivité à une FSH, un syndrome ovarien polykystique, une réserve ovarienne diminuée et une infertilité liée à l'âge.

12. Composition à utiliser selon la revendication 10, dans laquelle la femme mammifère est un être humain.

13. Composition à utiliser selon la revendication 10, dans laquelle le traitement comprend en outre une transplantation des follicules activés obtenus par le procédé selon les revendications 2 ou 4 à un receveur *in vivo.*

14. Composition à utiliser selon la revendication 13, dans laquelle le traitement comprend en outre l'administration d'un agoniste de LH audit receveur après implantation.
